(19) Europäisches Patentamt / European Patent Office / Office européen des brevets

(11) **EP 4 137 571 B1**

(12) **EUROPEAN PATENT SPECIFICATION**

(45) Date of publication and mention
of the grant of the patent:
**16.10.2024   Bulletin 2024/42**

(21) Application number: **21191995.6**

(22) Date of filing: **18.08.2021**

(51) International Patent Classification (IPC):
**C12N 15/09** (2006.01)    **C12N 15/67** (2006.01)

(52) Cooperative Patent Classification (CPC):
**C12N 15/09; C12N 15/67; C12N 2830/00**

(54) **BURDEN-ADDICTED PRODUCTION STRAINS**

LASTABHÄNGIGE PRODUKTIONSSTÄMME

SOUCHES DE PRODUCTION DÉPENDANTES DE LA CHARGE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**22.02.2023   Bulletin 2023/08**

(73) Proprietor: **Enduro Genetics ApS
2200 København N (DK)**

(72) Inventors:
• **FALKENBERG, Kristoffer Bach
2200 København N (DK)**
• **KÜHN, Johannes
2200 København N (DK)**
• **MUNCK, Christian
2200 København N (DK)**
• **RUGBJERG, Peter
2200 København N (DK)**
• **TARP LOGIS, Martin
2200 København (DK)**

(74) Representative: **Guardian
IP Consulting I/S
Diplomvej, Building 381
2800 Kgs. Lyngby (DK)**

(56) References cited:
• **CERONI FRANCESCA ET AL: "Burden-driven
feedback control of gene expression", NATURE
METHODS, vol. 15, no. 5, 1 May 2018 (2018-05-01),
New York, pages 387 - 393, XP055879792, ISSN:
1548-7091, Retrieved from the Internet
<URL:https://www.nature.com/articles/nmeth.46
35.pdf> DOI: 10.1038/nmeth.4635**

• **PASINI MARTINA ET AL: "Using promoter
libraries to reduce metabolic burden due to
plasmid-encoded proteins in recombinant
Escherichia coli", NEW BIOTECHNOLOGY, vol.
33, no. 1, 1 January 2016 (2016-01-01), NL, pages
78 - 90, XP055879818, ISSN: 1871-6784, DOI:
10.1016/j.nbt.2015.08.003**

• **BORKOWSKI OLIVIER ET AL: "Overloaded and
stressed: whole-cell considerations for bacterial
synthetic biology", CURRENT OPINION IN
MICROBIOLOGY, vol. 33, 2 August 2016
(2016-08-02), pages 123 - 130, XP029782245,
ISSN: 1369-5274, DOI: 10.1016/J.MIB.2016.07.009**

• **FRANCESCA CERONI ET AL: "Quantifying
cellular capacity identifies gene expression
designs with reduced burden", NATURE
METHODS, vol. 12, no. 5, 1 May 2015 (2015-05-01),
New York, pages 415 - 418, XP055626094, ISSN:
1548-7091, DOI: 10.1038/nmeth.3339**

• **GAO CONG ET AL: "Genetic Circuit-Assisted
Smart Microbial Engineering", TRENDS IN
MICROBIOLOGY, ELSEVIER SCIENCE LTD.,
KIDLINGTON, GB, vol. 27, no. 12, 15 August 2019
(2019-08-15), pages 1011 - 1024, XP085902234,
ISSN: 0966-842X, [retrieved on 20190815], DOI:
10.1016/J.TIM.2019.07.005**

• **JUNG SEUNG-WOON ET AL: "Recent advances
in tuning the expression and regulation of genes
for constructing microbial cell factories",
BIOTECHNOLOGY ADVANCES, ELSEVIER
PUBLISHING, BARKING, GB, vol. 50, 8 May 2021
(2021-05-08), XP086693021, ISSN: 0734-9750,
[retrieved on 20210508], DOI:
10.1016/J.BIOTECHADV.2021.107767**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 137 571 B1

Remarks:

The complete document including Reference Table(s) and the Sequence Listing(s) can be downloaded from the EPO website

**Description**

**FIELD OF THE INVENTION**

The invention provides a microbial production cell for synthesis of a product, further comprising a burden-addiction genetic circuit whose expression confers a selective growth and/or survival advantage on those cells that synthesize the product; while limiting proliferation of non/low-productive escaper cells.

**BACKGROUND OF THE INVENTION**

An increasing share of the world's chemical production relies on microorganisms that are genetically engineered to function as cell factories, and tailor-made for the biosynthesis of a given molecule. Production processes, employing these cell-factories, are typically initiated from a starter culture of a small number of cells of a production organism, which go through a phase of growth and expansion of cell numbers in large fermentation tanks (up to 1,000,000 L volume). In some setups, production of a given molecule proceeds both during the growth phase and during a subsequent period (batch and fed-batch cultures); and alternatively, production may be continuous. A chemostat fermenter allows a production organism to be grown in a fermentation broth that is constantly diluted, thus tapping product and cells from the culture, while replenishing with fresh nutrient medium. On an industrial scale, such production processes may continue operation for 1-2 months before starting a new culture in a clean tank. The fermentation processes and equipment used in this industry are very similar, both for the production of a wide range of commodity small molecules and for therapeutic proteins, and consequently these processes are subject to similar problems. In other applications, microbial cells may be engineered to produce a non-native molecule that confers a commercial advantage in the environment in which the cell is cultured.

Cell factories engineered to allocate finite metabolic resources to the biosynthesis of a given molecule are subject of an unnatural load; commonly reflected in slower growth. This load places a selective pressure for cells unable to synthesize the product molecule (non-producing or lower-producing), this problem particularly arising when the production run is for extended periods of time (e.g. in chemostats). Such non-producing cells within an industrial fermentation are highly undesirable, since they consume nutrients, oxygen and space. Since lower- and non-producing cells grow faster, they have a strong selective advantage over producing cells. In a growing cell culture, such improvements in fitness can lead to significant out-competition of the producing cells over time. This drift from the optimal production state is an eventual reason for discarding the fermentation broth and spending resources on cleaning, sterilization, not to mention nutrients, to replenish the fermentation tank with new, producing organisms. Such non-producing cells originate from genetic mutations that arise in the cells of an original producing organism undergoing many growth divisions.

Since the occurrence of genetic mutations and non-genetic adaptation in cells of a production organism that lead to a decline in product formation by the cells during a production run, cannot be avoided, there is a need for methods for eliminating or slowing the growth of non-producing cells in the production. Preferably, such methods of elimination are sufficiently effective that they prevent the observed drift from the production state, and thereby prolong the life-time of an industrial fermentation.

The present invention addresses the problem of how to deprive non-producing members of a cell factory with a fitness advantage; such as to delay their proliferation amongst members of a productive cell factory over time, and thereby improve the productivity of the cell factory.

**SUMMARY OF THE INVENTION**

A first aspect of the invention provides a microbial production cell genetically engineered to synthesize a product, wherein said cell further comprises:

- a first essential gene operably linked to a first burden-sensing promoter, and

- a second essential gene operably linked to a second burden-sensing promoter

wherein said first burden-sensing promoter is heterologous with respect to said first essential gene, and said second burden-sensing promoter is heterologous with respect to said second essential gene; wherein synthesis of the product confers a burden and/or fitness cost on said cell; and wherein expression of said first essential gene is up-regulated when said first burden-sensing promoter is induced by said burden and/or fitness cost relative to a basal level expression of said essential gene when said first burden-sensing promoter is not induced, and expression of said second essential genes is up-regulated when said second burden-sensing promoter is induced by said burden and /or fitness cost relative to a basal level expression of said second essential gene when said second burden-sensing promoter is not induced.

A second aspect of the invention provides a method of product biosynthesis comprising the steps of:

- providing at least one microbial production cell of the invention,
- introducing the at least one cell into a cultivation medium comprising substrate for production of said product,
- recovering said product.

A third aspect of the invention provides for a use of a first and a second essential gene operably linked to a first and a second burden-sensing promoter, respectively, to enhance product yield of a cultured population of microbial production cells arising from a single cell following at least 50 generations; wherein said first burden-sensing promoter is heterologous with respect to said first essential gene, and said second burden-sensing promoter is heterologous with respect to said second essential gene; wherein production of the product confers a burden on said cell; and wherein expression of said first essential gene is up-regulated when said first burden-sensing promoter is induced by said burden relative to a basal level expression of said first essential gene when said first burden-sensing promoter is not induced, and expression of said second essential gene is up-regulated when said second burden-sensing promoter is induced by said burden relative to a basal level expression of said second essential gene when said second burden-sensing promoter is not induced.

A fourth aspect of the invention provides for a use of a microbial production cell of the invention for producing a biosynthetic product.

A further aspect of the invention provides a microbial production cell genetically engineered to synthesize a product, wherein said cell further comprises:

- an essential gene operably linked to a burden-sensing promoter

wherein said burden-sensing promoter is heterologous with respect to said essential gene; wherein synthesis of the product confers a burden and/or fitness cost on said cell; wherein expression of said essential gene is up-regulated when said burden-sensing promoter is induced by said burden and/or fitness cost relative to a basal level expression of said essential gene when said burden-sensing promoter is not induced; and wherein said microbial production cell belongs to a genus selected from among Bacillus, Corynebacterium, and Aspergillus.

## DESCRIPTION OF THE INVENTION

### Brief description of the figures:

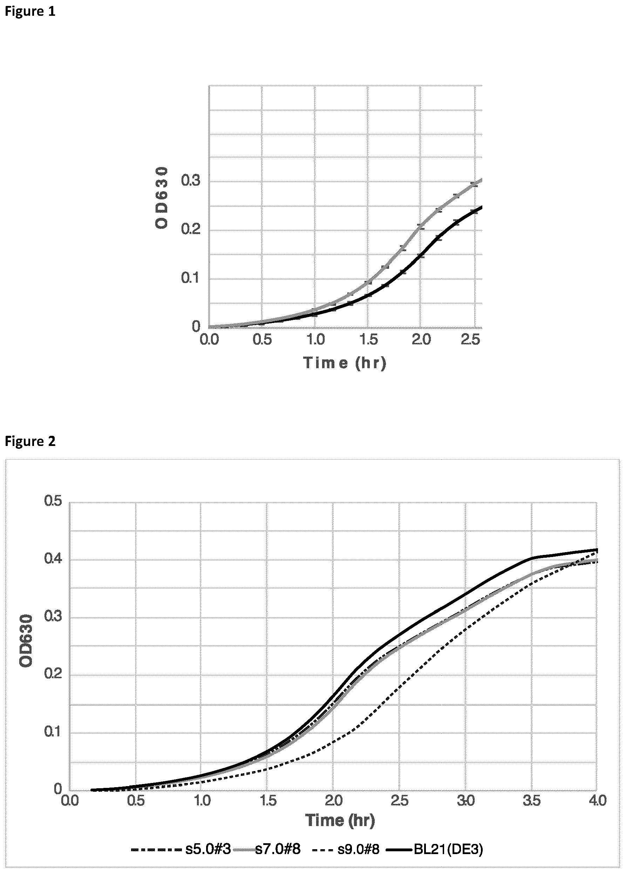

**Figure 1:** Graph showing growth (measured as optical density: OD $630_{bm}$) over time of cells of the parent strain *E. coli* BL21(DE3) (grey line) and cells of the parent strain transformed with a hGH-GFP production plasmid pEG34 (black line; pEG34). Growth of four replicate cultures of each strain was measured under hGH-GFP expression inducing conditions; error bars depict standard error of the mean (n = 4).

**Figure 2:** Graph showing growth (measured as optical density: OD $630_{bm}$) over time of cells of the parent strain *E. coli* BL21(DE3) (black line) and cells of the non-producing burden-addicted *E. coli* strains s5.0#3, s7.0#8 and s9.0#8 derived from the parent strain (as indicated; wherein essential genes *folP-glmM* are controlled by $p_{yccV}$, $p_{ycjX}$ and $p_{mutM}$ respectively). The measured growth rate is based on an average of four replicate cultures (n = 4).

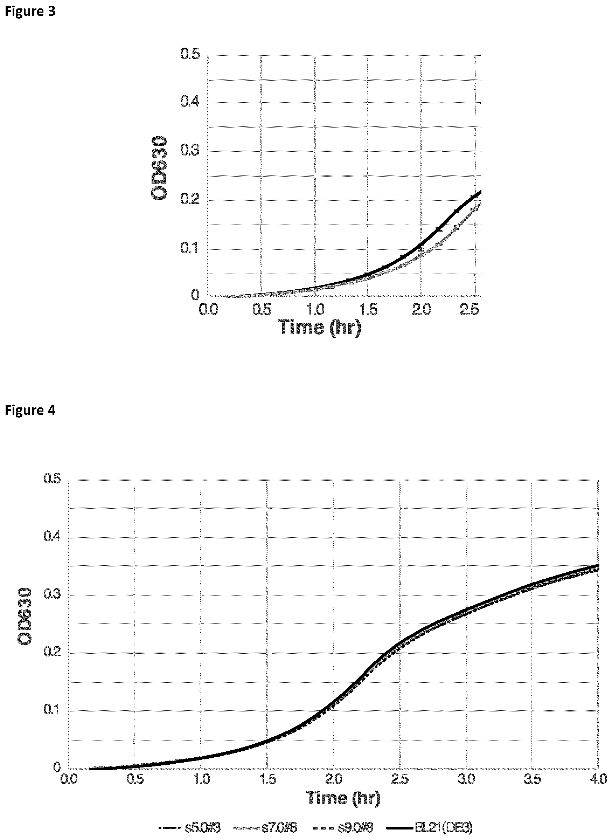

**Figure 3:** Graph showing growth (measured as optical density: OD $630_{bm}$) over time of cells of the burden-addicted strain s9.0#8 harboring either the hGH-GFP production plasmid pEG34 (black line) or the control plasmid pEG0 (grey line). Growth of four replicate cultures of each strain was measured under hGH-GFP expression inducing conditions; error bars depict standard error of the mean (n = 4).

**Figure 4:** Graph showing growth (measured as optical density: OD $630_{bm}$) over time of cells of the parent strain *E. coli* BL21(DE3) (black line), and cells of three burden-addicted strains: s5.0#3, s7.0#8 and s9.0#8 harboring and expressing the hGH-GFP production plasmid pEG34 (as indicated). The measured growth rate, measured under hGH-GFP expression inducing conditions, is based on an average of four replicate cultures (n = 4).

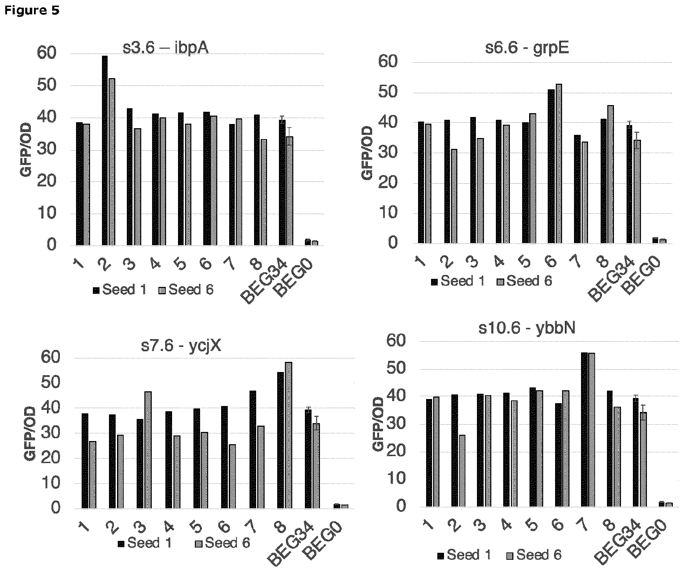

**Figure 5:** Histogram of GFP/OD values (Y-axis) measured during the first and sixth culture transfer (seed 1 and 6) of cultures of the hGH-GFP producing strains: s3.6 ($p_{ibpA}$), s6.6 ($p_{grpE}$), s7.6 ($p_{ycjX}$), and s10.6 ($p_{ybbN}$) comprising the respective burden-sensing promoters and one of four variant RBS coding sequences, driving expression of the essential gene, *folP-glmM*. Cultures of non-burden addicted *E. coli* strain BEG34 and the parent strain *E. coli* BL21(DE3), BEG0, are included as controls. Measurements are performed on cultures grown under hGH-GFP expression inducing conditions. Error bars depict standard error of the mean (n = 4).

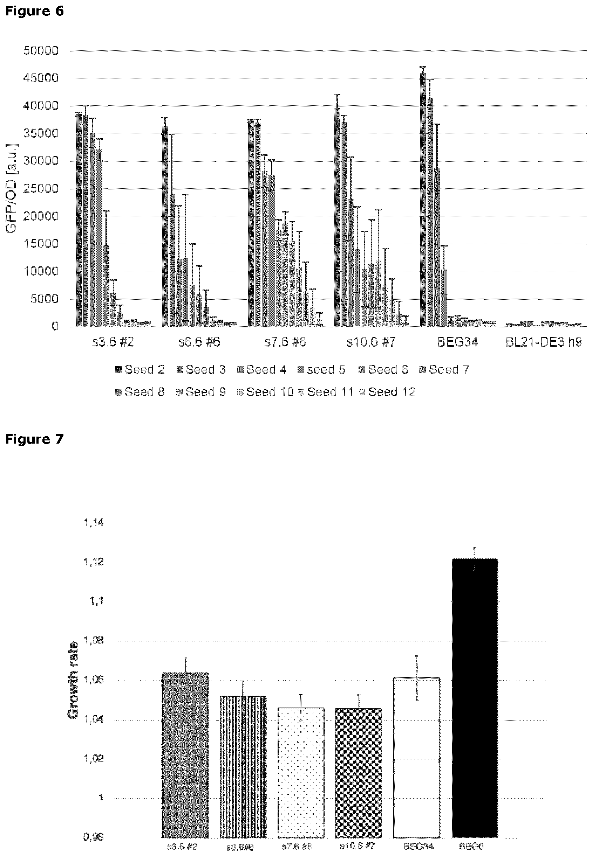

**Figure 6:** Histogram of GFP/OD values (Y-axis) measured after serial culture transfers (seed 1 to 12) of cultures of the hGH-GFP producing strains: s3.6#2 ($p_{ibpA}$), s6.6#6 ($p_{grpE}$), s7.6#8 ($p_{ycjX}$), and s10.6#7 ($p_{ybbN}$) comprising the respective burden-sensing promoters and one of four variant RBS coding sequences, driving expression of the essential gene, *folP-glmM*. Cultures of non-burden addicted *E. coli* strain BEG34 and the parent strain *E. coli* BL21(DE3) are included as controls. Measurements are performed on cultures grown under hGH-GFP expression inducing conditions. Error bars depict standard error of the mean (n = 3).

**Figure 7:** Histogram of OD/min as a measure of the growth rate of burden-addicted hGH-GFP producing strains s3.6#2, s6.6#6, s7.6#8 and s10.6#7 compared to non-burden addicted hGH-GFP producing *E. coli* strain BEG34 and the parent strain *E. coli* BL21-DE3 (BEG0). The growth rate of the respective strains is based on 7 replicate cultures measured under hGH-GFP expression inducing conditions at seed 0; error bars denote standard error of the mean (n = 7).

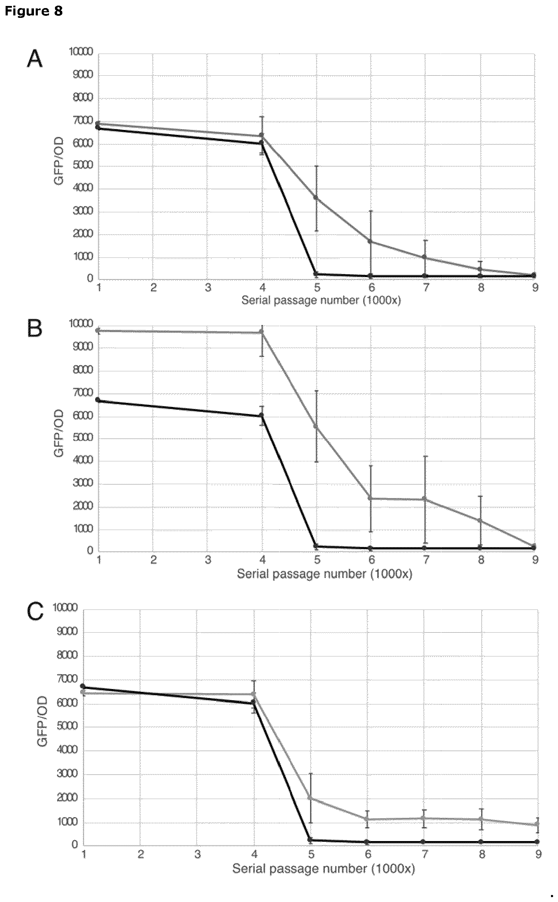

**Figure 8:** Graphs showing GFP/OD values (Y-axis) measured after serial culture transfers (seed 1, 4 to 9) of cultures of hGH-GFP producing strains, minus the values measured for an empty plasmid control BEG0-empty strain. The strains shown in: (A) s7.6#8 ($p_{ycjX}$), (B) s13.6#2 ($p_{fsxA}$), and (C) s13.6#2evo ($p_{fsxA}$), each comprise the respective burden-sensing promoter driving expression of the essential gene, *folP-glmM* (gray line) and a non-burden addicted hGH-GFP producing *E. coli* strain BEG34 (black line). Measurements are performed on cultures grown under hGH-GFP expression inducing conditions. Error bars depict standard error of the mean (n = 5).

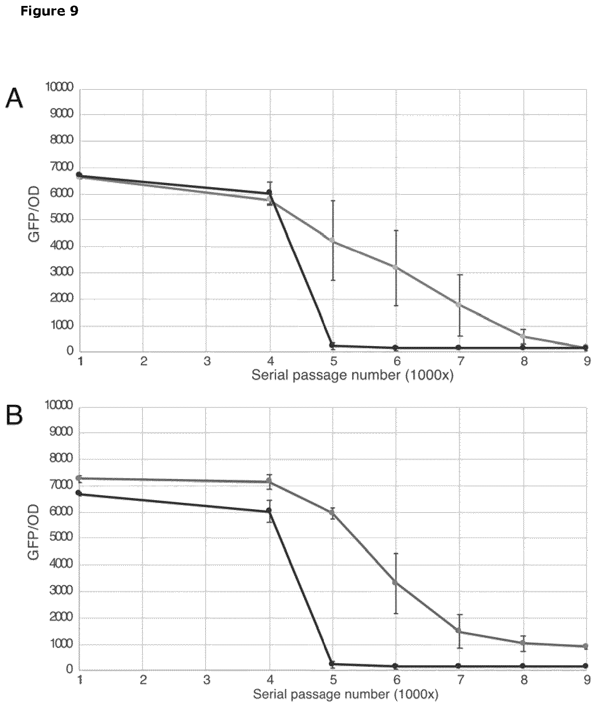

**Figure 9:** Graphs showing GFP/OD values (Y-axis) measured after serial culture transfers (seed 1, 4 to 9) of cultures of hGH-GFP producing strains, minus the values measured for an empty plasmid control BEG0-empty strain. The strains shown in: (A) s15.6.7 ($p_{rrnB}$) and (B) s16.6.6 ($p_{rrnE}$), each comprise the respective burden-sensing promoter driving expression of the essential gene, *folP-glmM* (gray line) and a non-burden addicted hGH-GFP producing *E. coli* strain BEG34 (black line). Measurements are performed on cultures grown under hGH-GFP expression inducing conditions. Error bars depict standard error of the mean (n = 5).

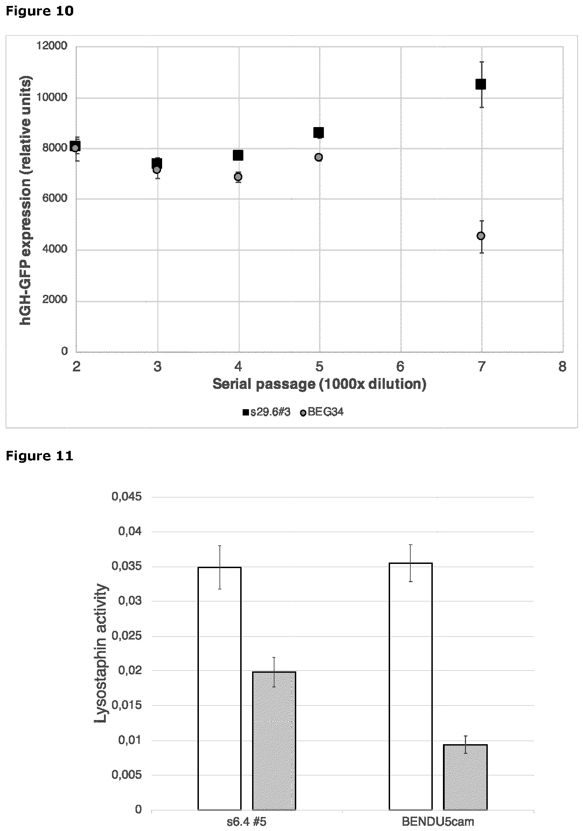

**Figure 10:** Graph showing relative hGH-GFP production (Y-axis) measured after serial culture transfers (seed 2 to 5, and 7) of cultures of a hGH-GFP producing strain, s29.6#3, minus the values measured for a parallel empty plasmid control BEG0 strain. Strain s29.6#3 comprises the osmotic stress sensing promoter $p_{poxB}$ driving expression of the essential gene, *folP-glmM* (black squares), compared to non-burden addicted hGH-GFP producing *E. coli* strain BEG34 (gray dots). Serial passage 7 was analyzed after 72 h. Measurements are performed on cultures grown under hGH-GFP expression inducing conditions. Error bars depict standard error of the mean (n = 5 for s29.6#3 and n = 8 for BEG34).

**Figure 11:** Histogram showing *S. aureus* lysis rate per minute, normalized to *E. coli* culture OD, as a measure of the secreted lysostaphin production rate of cultures of lysostaphin-producing strains s6.4#5 comprising the burden sensing promoter $p_{grpE}$ and one of four variant RBS coding sequences driving expression of the essential gene, *folP-glmM,* compared to non-burden addicted lysostaphin producing *E. coli* strain BENDU5cam. The rate was measured on cultures grown under lysostaphin expression inducing conditions after serial culture transfers (seed 1 and 7). Error bars depict standard error of the mean (n = 3).

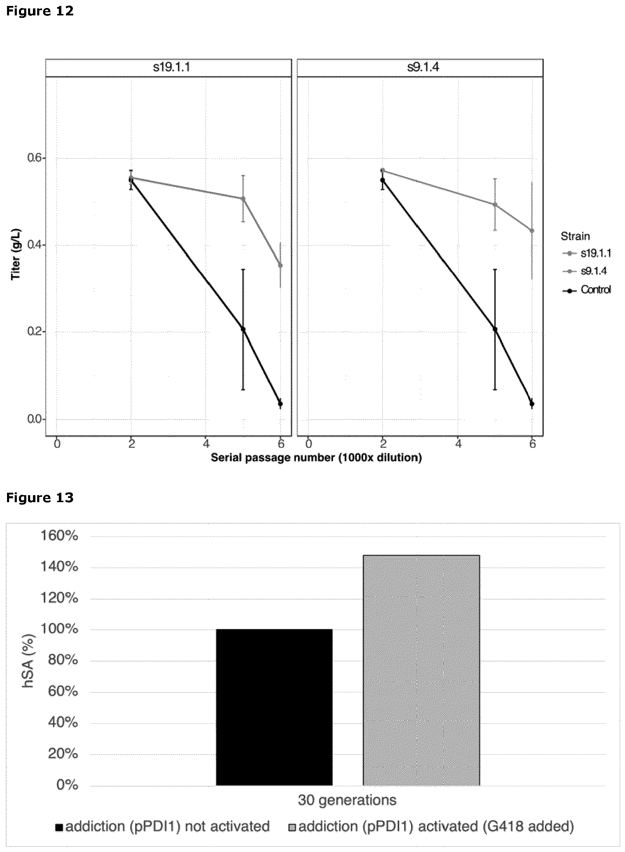

**Figure 12:** Graph showing mevalonic acid titer (g/L) synthesized by cultures of the burden-addicted mevalonic acid-producing strains s19.1#1($p_{cspD}$) and s9.1.4($p_{mutM}$), measured after serial culture transfers (seed 2, 5 and 6) compared to the parent non-burden addicted mevalonic acid-producing *E. coli* strain BL21(DE3)-pMevT. The burden sensing promoters, $p_{cspD}$ and $p_{mutM}$, are each combined with one of four variant RBS coding sequences driving expression of the essential gene, folP-glmM. Measurements are performed on cultures grown under MevT expression inducing conditions. Error bars depict standard error of the mean (n = 3).

**Figure 13:** Bar plot showing human serum albumin (hSA) production titers of cultures of *Pichia pastoris* strain EGS31 comprising a genomically-integrated copy of a cDNA version of the hSA coding *ALB1* gene under control of the *AOX1* promoter, and a *PDI1* promoter operably linked to the *kanMX* selection gene. The cultures were grown under either selecting conditions (addiction activated by 750 μg/mL G418) or non-selecting conditions (addiction not activated). The hSA titer of the 'addiction not activated' culture is set to 100%, and the titer of the 'addiction activated culture is shown as a percent thereof.

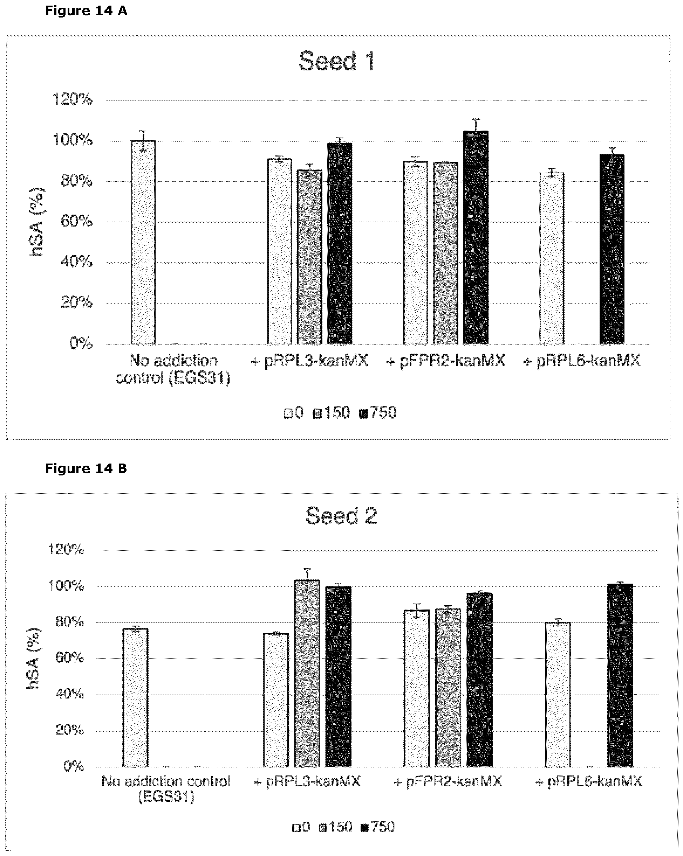

**Figure 14 A and B:** Bar plot showing human serum albumin (hSA) production titers of cultures of burden-addicted versions of the *Pichia pastoris* strain EGS31 each comprising a genomically-integrated copy of a cDNA version of the hSA coding ALB1 gene under control of the *AOX1* promoter and a *kanMX* selection gene operably fused to a burden responsive promoter *FPR2,* or *RPL3* or *RPL6A*. The cultures were grown under either non-selecting conditions (addiction not activated) or under selecting conditions (addiction activated with either 150 or 750 μg/mL G418) for a period of about 30 cell divisions ([A] Seed 1), or after a further 10 cell divisions ([B] Seed 2). Average hSA titers are shown in percent of the titer in the seed 1 'EGS31' culture. Error bars depict standard error of the mean (n = 4).

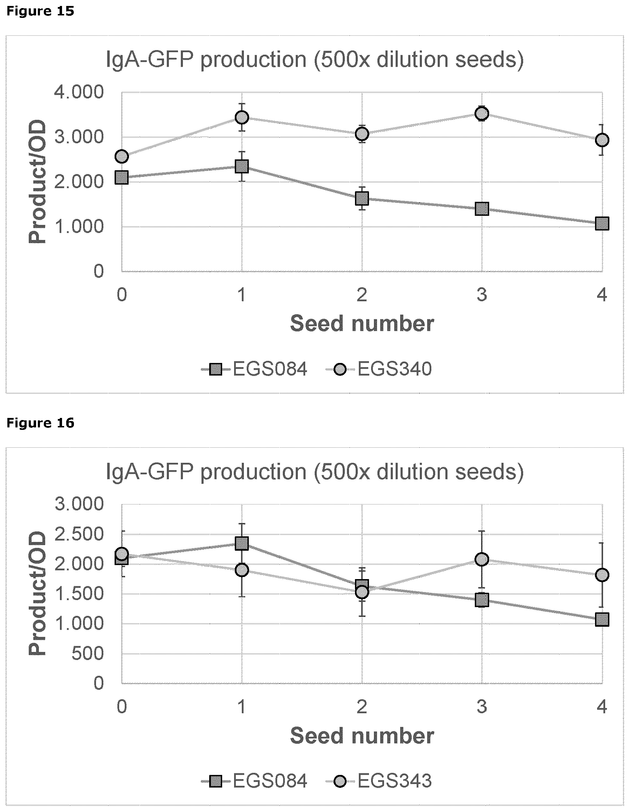

**Figure 15:** Comparison of IgA-GFP production of burden-addicted EGS340 and parental EGS84 in long-term cultivation. Demonstration of burden addiction in *Bacillus* to sustain production using the IgA production burden-sensing *perR* promoter to regulate transcription of the essential *iscU* gene operon (*sufC-sufD-sufS-sufU-sufB*). Comparison in EGS340 and EGS84 during serial-passaging experiment at 30 deg. C in which a 500x dilution seed is transferred to new culture medium every 24 hours. Points are averages and error bars show s.e.m. (n = 7).

**Figure 16:** Comparison of EGS343 and EGS84 in long-term cultivation. Demonstration of burden addiction in *Bacillus subtilis* to sustain production using the *perR* promoter to regulate transcription of the essential *accC* gene operon (*accB-accC-yhqY*). Comparison of IgA-GFP production in EGS343 and EGS84 during serial-passaging experiment at 30 deg. C in which a 500x dilution seed is transferred to new culture medium every 24 hours. Points are averages and error bars show s.e.m. (n = 7).

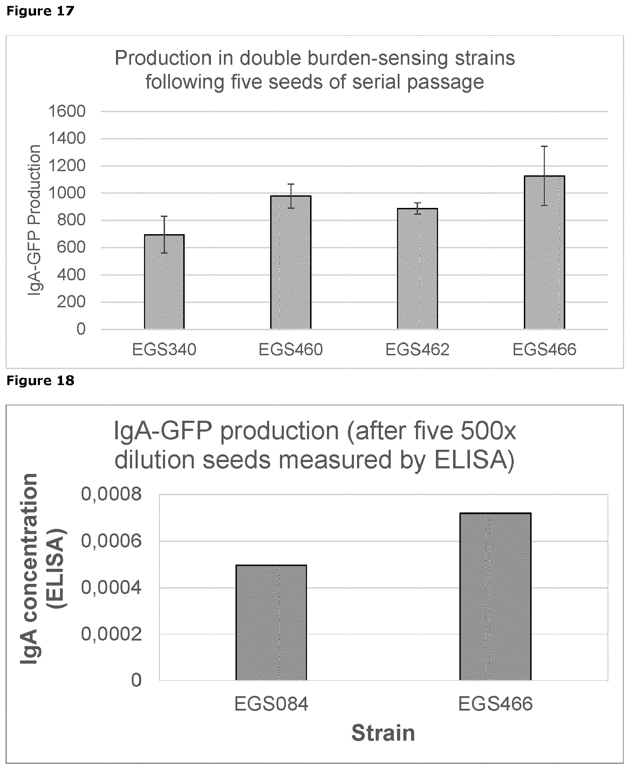

**Figure 17:** Use of several burden-sensors to simultaneously regulate different essential genes and thereby sustain production of a heterologous protein in *Bacillus subtilis.* Comparison of IgA-GFP production strains following five serial passages (500x dilution seeds) corresponding to approximately 75 generations. The single burden-sensor strain EGS340 carries only the $p_{perR}$-based burden sensor controlling transcription of the *iscU* essential gene operon (EGS340). EGS340 is compared to derived strains in which transcription of the *accC* essential gene operon is also regulated by respectively a $p_{ctsR}$-based burden sensor (EGS460), a $p_{dnaK}$-based burden sensor (EGS462), and a $p_{brcA}$-based burden sensor (EGS466). Bars show averages and error bars show s.e.m. (n = 4).

**Figure 18:** Comparison of relative IgA-GFP levels measured by IgA specific ELISA following approx. 75 generations of cell cultivations in the non-burden addicted control strain EGS084 and the double-burden addicted derived strain EGS466 (based on a $p_{perR}$ controlling the *iscU* essential gene operon and a $p_{hrcA}$ controlling the *accC* essential gene operon).

**Abbreviations, terms and definitions:**

**Burden** refers to the cellular state of a microbial cell (such as a cell genetically engineered to synthesize a product)during production of a product under production conditions, and which results in a fitness cost attributable to the synthesis of said product, in particular in a cell that exhibits high level efficient synthesis of the product. Fitness cost is a measure of said burden that can be quantified by measuring the percent reduction in the maximum exponential growth rate of the cell (measured along the growth curve) during synthesis of the product under production conditions as compared to a parent microbial cell incapable of said synthesis when grown under comparable production conditions.

**Burden-sensing promoter** refers to a promoter that is induced by said 'burden', and when induced, said burden-sensing promoter can upregulate the expression of a gene to which it is operably linked in a microbial production cell, when cultured under production conditions as compared to a mutant derivative of said microbial production cell that synthesizes essentially none, or at least 50 % less, of the intended product of the production cell. Such mutant derivative includes a non-productive escape mutant isolated following long-term cultivation of a population of cells derived from said production cell. Non-limiting examples (see Table 2 for details) of burden-sensing promoters include promoters of the *E. coli* genes *htpG, ibpA, clpB, yccV, grpE, ycjX, ldhA, mutM, ybbN, prlC, groES, fxsA, htpX, rrnB, rrnE, cspD, katE, xthA, uspE, gadB, ahpC, katG, grxA, oxyS, poxB, trxC* and their homolog versions in other gram-positive and gram-negative bacteria; for *S. cerevisiae* non-limiting examples of burden-sensing promoters include promoter of the genes KAR2, PDI1, SAA1, FPR2, RPL3, RPL6A, RPL28, OGG1, RAD51, RAD54; for Bacillus non-limiting examples of burden-sensing promoters include promoter of the genes *groES, ctsR, dnaK, perR, hrcA,*

*spx, sigB, yflT* and their homolog versions; for Corynebacterium non-limiting examples of burden-sensing promoters include promoter of the genes *groES, kata, cplX, mutM* and their homolog versions; for Aspergillus non-limiting examples of burden-sensing promoters include promoter of the genes *bipA, clxA* and their homolog versions. Burden-sensing promoters may also be hybrids, scrambled or truncated versions of such natural promoters as long as such promoters still maintain the response to burden.

**Burden-addicted microbial cell** refers to a microbial cell engineered to comprise a genetic circuit comprising at least one 'burden-sensing promoter' operably linked to an essential gene of the cell. Preferably said cell has a genetic circuit comprising two, namely a first essential gene operably linked to a first burden-sensing promoter, and a second essential gene operably linked to a second burden-sensing promoter wherein said first burden-sensing promoter is heterologous with respect to said first essential gene, and said second burden-sensing promoter is heterologous with respect to said second essential gene

**Burden-addicted microbial production cell** refers to microbial cell genetically engineered to synthesize a desired product, said cell being further genetically engineered to comprise a genetic circuit comprising said at least one, preferably two, 'burden-sensing promoter' operably linked to an 'essential gene' of the cell, as defined above. The desired product may be a protein encoded by a nucleic acid molecule in said engineered microbial cell, or a product of a heterologous pathway encoded by one or more nucleic acid molecules to be expressed by the cell.

**Non-burden addicted microbial production cell** is considered to be a "parent" cell of the burden addicted microbial production cell. It is a microbial production cell comprising and expressing one or more genes encoding the product or encoding a metabolic pathway for biosynthesis of the product, but lacking the genetic circuit comprising said at least one, preferably two, burden-sensing promoter operably linked to an essential gene as disclosed in the present invention. The at least one, preferably two, essential genes in this non-burden addicted microbial production cell wherein each is operably linked to its native promoter.

**Desired products** of the invention include - but are not limited to - a product that, when synthesized by the genetically engineered microbial production cell, conveys a fitness cost (burden) upon the production cell. Non-limiting examples of such product include an organic acid, terpenoid, isoprenoid, polyketide, alcohol, sugar, vitamin, aldehyde, carboxylic acid, fatty acid, amino acid, peptide, enzyme, therapeutic protein and precursors thereof, such as human growth hormone, insulin, glucagon-like peptide-1, a monoclonal or polyclonal antibody, a single-fragment antibody, and a nanobody. Examples further include proteins naturally found in eggs such as ovalbumin, or milk such as casein, lactadherin, alpha-lact lactoferrin, secreted immunoglobulin A and G, a secretory component. Examples of an enzyme includes an amylase, lipase, protease, barnase, β-galactosidase, crystal protein, cutinase, PETase, and laccase as well as a carbohydrate active enzyme such as a xylanase, lichenase, cellulase, lytic polysaccharide monooxoygenase, and a pectase.

**Essential gene** (or essential gene operon) refers to gene(s) in the microbial production cell which, if down-regulated, lead to reduction in the growth rate of the microbial production cell under production conditions. Preferably an essential gene is essential for growth irrespective of the nutrient composition of these production conditions, whereby sufficient expression of such essential genes to support cell growth would not be dependent on the presence or absence of specific inhibitors or nutrients provided under the production conditions . Non-limiting examples of *E. coli* essential genes during standard laboratory conditions include *folP-glmM, glmM, murI, asd, thyA, rpoD, nusG, rpsU, accD, degS, fldA, ftsN, hflB, lolA, mraY, mreD, murA, murB, murF, nadD, rplV, rpsG,* and homologues thereof. Non-limiting examples *for Bacillus* include *iscU operon (sufC-sufD-sufS-sufU-sufB), accC operon (accB-accC-yhqY), glmM, ylaN, infA, dapA* and homologues thereof. Non-limiting examples for *Saccharomyces* include, *FOL1, MED7, RRP40, NOPS, PGI1, NEP1, URA3 LEU2, TRP1, HIS3* and homologues thereof. Non-limiting examples for Aspergillus include *ARG3, adeA,* and homologues thereof: Non-limiting examples for Corynebacterium include *alr, glmM,* and homologues thereof: For example, suitable genes encode enzymes responsible for synthesizing cell wall constituents. Further, an essential gene used in the context of this invention preferably neither encodes a desired product to be expressed by the production strain; nor encodes a protein that facilitates synthesis of a desired product or intermediate thereof in a heterologous pathway to be expressed by the production strain.

**Conditional essential gene** is one that allows the burden addiction system in the microbial production cell to be "activated" by culturing the cells under the condition of essentiality such as added antibiotic or removed nutrient.

**Essential gene basal expression level** is the level of transcription of each 'essential gene' (as defined above) that is operably linked to a 'burden-sensing promoter' in a burden-addicted microbial production cell, when the 'burden-sensing promoter' is not induced. The burden sensing promoter to which the essential gene is operably linked is heterologous with respect to said essential gene, in the sense that the promoter is not the native promoter of said essential gene (even though the promoter may be present in the same genome), and is thus not found operably linked to said essential gene in nature. Said basal (i.e. un-induced) level expression of an essential gene is a level sufficient to support growth of a cell under production conditions (or during an exponential growth phase) at a level equal to or less than 10, 20, 50, 90 or 95% of the growth rate of a corresponding cell wherein each said essential gene is operably linked to its native promoter. A lowered growth rate due to basal level essential gene expression in those cells where the burden sensing promoter is not activated constitutes a selective disadvantage for non-productive cells, for example non-productive mutants arising during product production.

**Fitness cost:** is quantified by comparing the maximum exponential growth rate (when measured along the growth curve) of a non-burden addicted microbial production cell or cell culture thereof (i.e. lacking burden-sensing promoters operably linked to the at least one, preferably two essential genes), but comprising and expressing one or more genes encoding the product or encoding a metabolic pathway for biosynthesis of the product) relative to the maximum exponential growth rate of (i) a parent microbial cell (or cell culture thereof) either devoid or incapable of expressing gene(s) encoding the product or metabolic pathway for biosynthesis of the product, from which the microbial production cell was derived (or (ii) an escape cell derived from the non-burden addicted microbial production cell, wherein said escape cell produces less than 50% of the product compared to the non-burdened addicted microbial production cell), when grown under comparable production conditions. The maximum exponential growth rate of the respective cells or cell cultures thereof is to be measured at the start of production following introduction of the respective cells or cell cultures thereof into a cultivation medium comprising substrate for production of said product.

**Production conditions** refers to specific cultivation conditions to be used during production and can relate to medium and/or other culture conditions (e.g. temperature, pH, stirring, aeration, etc) for production of a desired product by the microbial production cell.

**Ribosomal binding site** (RBS), translation initiation region, or translational strength element refers to the genetic region of the 5' untranslated region that control the translation strength of a particular messenger RNA.

## Detailed description of the invention:

### I. A burden-addicted microbial production cell

A first aspect of the present invention provides a microbial production cell genetically engineered to synthesize a product, wherein said cell further comprises:

a. an essential gene operably linked to a burden-sensing promoter,

wherein said promoter is heterologous with respect to said essential gene; wherein production of the product confers a burden and/or fitness cost on said cell, and wherein expression of said essential gene is up-regulated when said burden-sensing promoter is induced by said burden and/or fitness cost relative to a basal level expression of said essential gene when said burden-sensing promoter is not induced.

In a further aspect of the present invention, the microbial production cell genetically engineered to synthesize a product comprises:

a. a first essential gene operably linked to a first burden-sensing promoter, and
b. a second essential gene operatively linked to a second burden-sensing promoter,

wherein said first burden-sensing promoters is heterologous with respect to said first essential genes, and said second burden-sensing promoters is heterologous with respect to said second essential genes;
wherein synthesis of the product confers a burden on said cell, and
wherein expression of said first essential genes is up-regulated when said first burden-sensing promoter is induced by said burden relative to a basal level expression of said first essential gene when said first burden-sensing promoter is not induced, and
expression of said second essential genes is up-regulated when said second burden-sensing promoter is induced by said burden relative to a basal level expression of said second essential gene when said second burden-sensing promoter is not induced.

In one embodiment, the first and second essential genes are different. In another embodiment, the first and second essential genes are identical. In a specific embodiment, the first and second essential genes are identical while the first and second burden-sensing promoters are different.

In one embodiment, the first and second burden-sensing promoters are different. In another embodiment, the first and second burden-sensing promoters are identical. In a specific embodiment, the first and second burden-sensing promoters are identical while the first and second essential genes are different.

A microbial production cell genetically engineered to synthesize a product is one that comprises one or more genes encoding the product or encoding a metabolic pathway for synthesis of the product, wherein optionally said one or more genes are operably linked to a constitutive or inducible promoter. In one embodiment, the one or more genes encoding the product or encoding a metabolic pathway for synthesis of the product may be recombinant.

According to one embodiment, said burden and/or fitness cost on said microbial production cell of the invention, is quantified by comparing the maximum exponential growth rate of the microbial production cell comprising one or more genes encoding the product or encoding a metabolic pathway for synthesis of the product, (but lacking burden-sensing promoters operably linked to the at least one preferably two essential gene), relative to the maximum exponential growth rate of a parent microbial cell lacking or incapable of expressing one or more genes encoding the product or encoding a metabolic pathway for synthesis of the product, and from which said microbial production cell was derived, where the respective cells are cultured under essentially identical production conditions. The burden and/or fitness cost on said microbial production cell preferably corresponds to a percent reduction in the quantified maximum exponential growth rate selected from among $\geq 5\%$, $\geq 10\%$, $\geq 15\%$, $\geq 20\%$, $\geq 25\%$, $\geq 35\%$ and $\geq 45\%$.

The burden-addicted microbial production cell according to the invention may be any prokaryotic or eukaryotic microorganism such as a bacterium, yeast, and filamentous fungus.

In one embodiment, the microbial production cell of the invention is a prokaryote. A non-exhaustive list of suitable bacteria is given as follows: a species belonging to the genus selected from among *Escherichia, Lactobacillus, Lactococcus, Corynebacterium, Bacillus, Acetobacter, Acinetobacter, Pseudomonas; Proprionibacterium, Bacteroides* and *Bifidobacterium.*

In another embodiment, the microbial production cell of the invention is a eukaryote, such as a yeast or fungus. In certain embodiments, the eukaryote can be a member of the genus *Saccharomyces, Komagataella* or *Aspergillus.*

A non-exhaustive list of suitable yeasts is given as follows: a yeast belonging to the genus of *Saccharomyces,* e.g. *S. cerevisiae, S. kluyveri, S. bayanus, S. exiguus, S. sevazzi, S. uvarum;* a yeast belonging to the genus *Kluyveromyces,* e.g. *K. lactis K. marxianus* var. *marxianus, K. thermotolerans;* a yeast belonging to the genus *Candida,* e.g. *C. utilis C. tropicalis, C. albicans, C. lipolytica, C. versatilis;* a yeast belonging to the genus *Pichia,* e.g. *P. stipidis, P. pastoris, P. sorbitophila,* or other yeast genera, e.g. *Cryptococcus, Debaromyces, Hansenula, Yarrowia, Zygosaccharomyces* or *Schizosaccharomyces.*

A non-exhaustive list of suitable filamentous fungi is given as follows: a filamentous fungus belonging to the genus of *Penicillium, Rhizopus, Fusarium, Fusidium, Gibberella, Mucor, Mortierella, Trichoderma Thermomyces, Streptomyces* and *Aspergillus.* More specifically, the filamentous fungus may be selected from *Fusarium oxysporum, A. niger, A. awamori, A. oryzae,* and *A. nidulans.*

The product(s) synthesized by the microbial production cell of the invention is one that incurs a burden on the cell and fitness cost reflected in a reduction in growth rate, such product(s) including amino acids, organic acids, terpenoids, isoprenoids, polyketides, alcohols, sugars, vitamins, aldehydes, carboxylic acids, fatty acids, peptides, enzymes, therapeutic proteins and their precursors such as human growth hormone, insulin, glucagon-like peptide-1, monoclonal and polyclonal antibodies, single-fragment antibodies and nanobodies. Examples further include proteins naturally found in eggs such as ovalbumin, or milk such as caseins, lactadherins, alpha-lactalbumin, osteopontin, lactoferrin, secreted immunoglobulin A and G, secretory components. Further examples of enzymes are amylases, lipases, proteases, barnases, β-galactosidases, crystal proteins, cutinases, PETases, and laccases as well as carbohydrate-active enzymes such as xylanases, lichenases, cellulases, lytic polysaccharide monooxoygenases, and pectases. In one embodiment the product is not a native product of the microbial production cell of the invention, and thus not produced by a parent cell from which the microbial production cell was derived. In one embodiment, the one or more genes encoding the product or encoding a metabolic pathway for synthesis of the product are heterologous with respect to the microbial production cell of the invention; where said one or more genes may be transgenes.

## II. Features of the microbial production cell of the invention

*II. i Fitness cost - production burden*

Heterologous expression of a desired product or pathways leading to a desired product in a microbial cell imposes a burden on the cell. Burden can also result from overproduction of a native product, e.g. in cells of microbial strains selected following mutagenesis. The burden may be described as a fitness cost which results in slower growth of the cell (Example 1; figure 1). This may be due to general and/or product-specific metabolic toxicities, increased use and/or depletion of potentially finite cell resources and metabolites, which a microbial cell experiences during the biosynthesis of a product of the invention under production conditions. Particularly during industrial scale fermentation, a combination of fitness cost and slower growth places a selective pressure on the population of cells in the fermenter, which over time leads to a decline in product yield due to an accumulation of non-productive cells resulting from genetic mutations or non-genetic variation (example 2, BEG34 in figure 6).

In the present invention, the presence and expression of one or more genes encoding a product or encoding a metabolic pathway for biosynthesis of a product in a microbial production cell confers a fitness cost (burden).

The fitness cost of producing a product on a microbial production cell is quantified by comparing the growth rate of a non-burden addicted microbial production cell (i.e. lacking at least one, preferably two, burden-sensing promoters operably linked to the essential genes, but comprising and expressing one or more genes encoding the product or encoding a metabolic pathway for biosynthesis of the product) relative to the growth rate of (i) a parent microbial cell from which the non-burden addicted microbial production cell was derived, wherein said parent microbial cells is devoid of gene(s) encoding the product or metabolic pathway for biosynthesis of the product, or (ii) an escape cell derived from the non-burden addicted microbial production cell, wherein said escape cell produces less than 50% of the product compared to the non-burdened addicted microbial production cell. The burden and/or fitness cost on said microbial production cell preferably corresponds to a percent reduction in the quantified growth rate selected from among $\geq 5\%$, $\geq 10\%$, $\geq 15\%$, $\geq 20\%$, $\geq 25\%$, $\geq 35\%$ and $\geq 45\%$, more preferably at least 5%.

Preferably quantification of the relative growth rates is performed on the respective microbial cells by measuring their maximum exponential growth rate when cultured under essentially identical conditions, these conditions being chosen to closely mimic those in which eventual large-scale fermentation is to take place. All uses of growth rate refers to the term specific growth rate.

*II.ii Essential gene*

A microbial production cell of the invention comprises at least one preferably two essential gene(s) encoding at least one preferably two protein(s), respectively, whose expression is required for cell growth and/or survival.

In one embodiment, the microbial production cell comprises a first and a second essential gene encoding a first and a second protein (where the first and second protein may be the same or different), respectively, wherein the expression of both said first and said second protein is required for cell growth and/or survival.

Preferably, the at least one preferably two essential genes and their expression do not indirectly cause a fall in production of the desired product by the production cell. Further, the essential gene(s) used in the context of this invention do not encode or lead to synthesis of a desired product or intermediate of a heterologous pathway to be expressed in the microbial production cell.

The at least one preferably two essential gene(s) are preferably non-conditional essential gene(s), such that expression of the gene(s) is essential for cell growth and/or survival irrespective of the composition of the growth medium or conditions in which the cell is cultured.

In one embodiment of the invention, when the production cell is a prokaryote, such as *E. coli,* the at least one preferably two non-conditional essential gene(s) are selected from *folP-glmM, glmM, murI, asd, thyA, usA, rpoD, nusG, rpsU, accD, degS, fldA, ftsN, hflB, lolA, mraY, mreD, murA, murB, murF, nadD, rplV* and *rpsG,* and homologues thereof.

In a further embodiment of the invention, when the production cell is a prokaryote, such as a *Bacillus* strain, the at least one preferably two non-conditional essential gene is selected from *iscU operon, accC operon, glmM, ylaN, infa,* and *dapA,* and homologues thereof.

In one embodiment of the invention, when the production cell is a prokaryote, such as a *Corynebacterium* strain, the at least one preferably two non-conditional essential gene is selected from *alr, glmM,* and homologues thereof

In one embodiment of the invention, when the production cell is a eukaryote, the essential gene when non-conditional is selected from *S. cerevisiae FOL1, MED7, RRP40, NOPS, PGI1, NEP1* and homologues thereof; and when conditional is selected from *S. cerevisiae URA3, LEU2, TRP1, HIS3* and homologues thereof.

In one embodiment of the invention, when the production cell is a filamentous fungi, such as Aspergillus, the non-conditional essential gene is selected from *ARG3, adeA,* and homologues thereof.

In one embodiment of the invention, the at least one preferably two essential genes are conditionally essential genes leading to the synthesis of a product required for auxotrophic growth, or a protein product required for resistance to growth inhibitors such as an antibiotic, specific toxin, protoxin, or the like.

When the at least one preferably two essential genes are conditionally essential then the composition of the production cell's growth medium/growth conditions must be adjusted, such as using growth medium lacking specific nutrients or supplemented with growth inhibitors.

In one further embodiment, the production cell may comprise a combination of a non-conditional and a conditional essential gene.

| Table 1 Non-exhaustive list of essential genes | | | |
|---|---|---|---|
| SEQ ID NO. | Gene[#1] | Gene encoded product(s) | |
| | *E. coli* | | Ecocyc/ Biocyc accession ID[#2] |
| 1 | folP-glmM | Operon of genes encoding dihydropteroate synthase and phosphoglucosamine mutase | EG50011 |
| 3 | glmM | phosphoglucosamine mutase | EG11553 |
| 5 | murI | glutamate racemase | EG11204 |
| 7 | asd | aspartate-semialdehyde dehydrogenase | EG10088 |
| 9 | thyA | thymidylate synthase | EG11002 |
| 11 | rpoD | RNA polymerase, sigma70 | EG10896 |
| 13 | nusG | transcription termination factor NusG | EG10667 |
| 15 | rpsU | 30S ribosomal subunit protein S21 | EG10920 |
| 17 | accD | acetyl-CoA carboxyltransferase subunit β | EG10217 |
| 19 | *degS* | serine endoprotease | EG11652 |
| 21 | *fldA* | flavodoxin 1 | EG10318 |
| 23 | *ftsN* | cell division protein FtsN | EG11529 |
| 25 | *hflB* | ATP-dependent zinc metalloprotease Fts H | EG11506 |
| 27 | *lolA* | outer membrane lipoprotein carrier protein | G6465 |
| 29 | *mraY* | 17hosphor-N-acetylmuramoyl-pentapeptide-transferase | EG10604 |
| 31 | *mreD* | cell shape determining protein MreD | EG10610 |
| 33 | *murA* | UDP-N-acetylglucosamine 1-carboxyvinyltransferase | EG11358 |
| 35 | *murB* | UDP-N-acetylenolpyruvoylglucosamine red uctase | G11205 |
| 37 | *murF* | D-alanyl-D-alanine-adding enzyme | EG10622 |
| 39 | *nadD* | nicotinate-mononucleotide adenylyltransferase | G6350 |
| 41 | *rplV* | 50S ribosomal subunit protein L22 | EG10882 |
| 43 | *rpsG* | 30S ribosomal subunit protein S7 | EG10906 |
| | *S. cerevisiae* | | SGD ID[#3] |
| 45 | *FOL1* | dihydroneopterin aldolase | SGD:S000005200 |
| 47 | *MED7* | mediator complex subunit MED7 | SGD:S000005495 |
| 49 | *RRP40* | exosome non-catalytic core subunit RRP40 | SGD:S000005502 |
| 51 | *MOPS* | Nucleolar protein required for 60S ribosomal subunit biogenesis | SGD:S000005504 |
| 53 | *PGI1* | glucose-6-phosphate isomerase | SGD:S000000400 |
| 55 | *NEP1* | 18S rRNA pseudouridine methyltransferase | SGD:S000004176 |
| 57 | *URA3* | orotidine-5'-phosphate decarboxylase | SGD:S000000747 |
| 59 | *LEU2* | 3-isopropylmalate dehydrogenase | SGD:S000000523 |
| 61 | *TRP1* | phosphoribosylanthranilate isomerase | SGD:S000002414 |

(continued)

| | | S. cerevisiae | | SGD ID[#3] |
|---|---|---|---|---|
| | 63 | HIS3 | imidazoleglycerol-phosphate dehydratase | SGD:S000005728 |
| | | **Bacillus** | | BL lotus tag[#4] |
| | 147 | glmM | Phosphoglucosamine mutase | BL02703 |
| | 149 | ylaN | Protein controllinq cell shape | BL02974 |
| | 151 | infA | Translation initiation factor | BL01028 |
| | 153 | dapA | Dihydrodipicolinate synthase | BL01208 |
| | 165 | sufC (in iscU operon) | | |
| | 167 | sufD (in iscU operon) | | |
| | 169 | sufS (in iscU operon) | | |
| | 171 | sufU (in iscU operon) | | |
| | 173 | sufB (in iscU operon) | | |
| | 175 | accB (in accC operon) | | |
| | 177 | accC (in accC operon) | | |
| | 179 | yghY (in accC operon) | | |
| | | **Corynebact erium[#3]** | | |
| | 181 | glmM | phosphocilucosamine mutase | G18NG-10145 |
| | 183 | air | Alanine racemase | G18NG-10150 |
| | | **Aspergillus [#5]** | | |
| | 185 | ARG3 / argB | Ornithine carbamoyltransferase | AO090023000856 |
| | 187 | adeA | Phosphoribosylaminoimidazole-succinocarboxamidesynthase | AO090020000395 |

[#1] An essential gene according to the invention is one encoding a protein that has at least 70%, 80%, 90%, 95% or even 100% amino acid homology with the protein encoded by the respective essential gene listed in the Table.
[#2] BioCyc/EcoCyc Database Collection is a large online collection of Pathway/Genome Databases - accessed through https://biocyc.org/
[#3] SGD is the "Saccharomyces Genome Database" - accessed through https://yeastgenome.org/
[#4] BL: accessed through https://www.genome.jp/kegg-bin/show_organism?org=bli
[#5] Aspergillusgenome.org

The essentiality of a gene can be determined by creating cells in which the respective gene is knocked-out, where a non-conditional gene knockout will result in a loss of cell viability, or failure to grow, irrespective of growth conditions; while a conditional gene knockout will result in a failure to grow or loss of cell viability depending on the composition medium/conditions of cultivation.
A suitable essential gene can be identified by performing a test assay that swaps the native promoter of the assayed essential gene for an inducible promoter, such as the L-arabinose inducible $p_{BAD}$ (in E. coli),), xylose-inducible

$p_{XYL}$ (in *Bacillus subtilis* or *Bacillus licheniformis*) or galactose inducible $p_{GAL}$ (in *S. cerevisiae* or *Pichia Pastoris*), or plac (in *E. coli* or *Lactobacillus*). In such an essential gene assay, an integration DNA construct comprising the inducible promoter is chromosomally integrated into a host microbial cell using standard methods. In the case of prokaryotic organisms, the integration DNA preferably harbors an RBS catalog (e.g., Table 3) to direct translation of the essential gene at different rates to account for different baseline expression levels of different promoters and different baseline required expression levels required of essential genes to support growth at the corresponding wildtype specific growth rate. After targeted integration of the integration DNA, integrant cells are plated on plates supplemented with permissive conditions (temperature or concentration of inducers: >0.9 % L-arabinose, > 0.8% xylose, >1% IPTG, >2% galactose). Cells are subsequently tested for their synthetic addiction to the inducer condition in standard growth curve assays, and suitable essential genes can be identified as genes for which inducer-dependent growth rates can be identified. Inducer-dependent growth can be observed as a reduced growth rate of > 5 percent in absence of inducing conditions. Suitable essential genes are characterized by conferring less than < 5 % reduction in exponential-phase specific growth rate during optimal inducer conditions in production-relevant cultivation medium.

Methods for identification of an essential gene are detailed in "Gene Essentiality - Method and Protocols" January 2015 [DOI 10.1007/978-1-4939-2398-4] which enables the skilled person to determine whether a gene is essential for microbial cell growth under specific growth conditions; including methods for mapping and identifying essential genes of *Campylobacter jejuni; Streptococcus sanguinis; Porphyromonas gingivalis; Escherichia coli; Leptospirosis; Mycobacterium tuberculosis; Pseudomonas aeruginosa;* and *Candida albicans*; primarily by screening transposon tagged libraries. Additionally, a variety of "computational tools" described therein enable the skilled person to directly predict and identify genes encoding essential proteins in a microbial genome, facilitated by the widespread availability of whole genome microbial sequences; and the structural features of the many known essential genes. Accordingly the skilled person is provided with both databases of essential genes; and various freely accessible on-line tools and algorithms for successfully and reproducibly identifying large numbers of essential genes in the genome of microbial cells without undue burden. It should further be noted that 82% of the essential genes in *Saccharomyces cerevisiae* encode essential proteins that are similar to a protein in another organism [Giaever G et al., 2002]; demonstrating that most essential genes, either known or identified in one micro-organism (e.g. yeast) will have a counterpart in other microorganisms - and could thus be identified in a microorganism of choice by a simple BLAST search.

*II.iii Burden-sensing promoter*

The microbial production cell of the invention comprises at least one preferably two burden-sensing promoter(s) operably linked to the at least one preferably two essential gene(s) encoding at least one preferably two protein(s), respectively, required for cell growth and/or survival, as described above. A burden-sensing promoter is one that is induced in a microbial production cell that is subject to a burden and/or fitness cost due to its production or over-production of a product. Typically, induction of the burden-sensing promoter will occur when a microbial cell's production of a product results in a reduction in the cell's growth rate that, in turn, is attributable to the fitness cost of production. Importantly, the at least one preferably two burden-sensing promoters, according to the invention, are induced by the "burden status" in the production cell resulting from production of a product, and not by the product *per se*. Since the use of the at least one preferably two burden-sensing promoter and essential gene to enhance productivity of a microbial production cell is largely independent of the product it is engineered to make, the burden-addiction conferred by the invention supports a wide range of microbial production cell applications.

Since the essential gene is operably linked to the burden-sensing promoter, its level of transcription and expression is only increased above a basal level when the burden-sensing promoter is induced. A suitable burden-sensing promoter is one that, when un-induced, permits a basal level of expression of the operably linked essential gene that is significantly limiting for, or prevents cell growth and/or survival. Basal level expression of essential gene is a level sufficient to support growth of a cell under production conditions (or during an exponential growth phase) at a level equal to or less than 10, 20, 50, 90 or 95% of the growth rate of a corresponding cell wherein said essential gene is operably linked to its native promoter. This basal level of expression of essential gene will be exerted in burden addicted cells when the burden-sensing promoters is not activated by the burden of efficient product synthesis, and thereby, the resulting lowered growth rate will constitute a selective disadvantage upon lack of product synthesis.

Cells comprising burden-sensing promoter in the un-induced state, will grow significantly slower than cells where the essential gene is operably linked to its native promoter (example 1, figure 2). By contrast, microbial production cells comprising the burden-sensing promoter linked to an essential gene exhibit a significantly increased growth rate when the cells synthesize the desired product as compared to cells not producing the product (example 1, figure 3). Furthermore, microbial production cells of the invention, despite the inherent burden of producing a product, can surprisingly achieve an initial growth rate that is indistinguishable from the growth rate of parent microbial cells, devoid of gene(s) encoding the product or metabolic pathway for biosynthesis of the product, and from which the microbial production cell was derived (example 1, figure 4). Hence under production conditions, a microbial production cell of the invention will have a selective advantage over an escape variant of the production cell that ceases to synthesize the product, and thereby depress the escape rate.

To increase production of desired products and further prolong the period during which the production cells are productive, more than one burden-sensing promoter may be suitable to use to promote growth of beneficial cell variants. By using two or more different burden-sensing promoters to addict a production cell, the highly productive cell variant can more uniquely be differentiated from less productive (genetic/non-genetic) cell variants that over time may arise from the same ancestral starting cell. For example, environmental factors e.g. resulting from outside stresses in a production culture may to some extent activate even less productive cells and thereby permit growth.

By using more than one burden-sensor to control essential genes, the transcriptional space that directs the imposed addiction-based selection regime can be further controlled. This may be important to limit potential cellular escape modes in which the burden addiction response of a single burden biosensor may permit sufficient growth to allow production declines.

Table 2 provides a non-exhaustive list of potential burden-sensing promoters.

In one embodiment, the burden-sensing promoter may be a native promoter, or a modified native promoter, with respect to the microbial production cell, while being heterologous with respect to the essential gene.

In one embodiment, the promoter contains native TF/sigma factor binding sites. Suitable burden-sensing promoters are those of the *E. coli* σ32 regulon since they are generally upregulated in response to over-expression of proteins and their intracellular aggregation. A non-exhaustive list of *E. coli* σ32 promoters is included herein in Table 2, while additional suitable promoters are identified by Nonaka et al, 2006.

In one embodiment, the burden-sensing promoter is a non-vegetative σ factor regulated promoter. In a preferred embodiment, the burden-sensing promoter is activated via the σ32 regulon, and can be selected from among promoters of the *E. coli* genes *htpG, ibpA, clpB, yccV, grpE, ycjX, ldhA, mutM, ybbN, prlC, groES, fxsA,* and *htpX.*

In one embodiment, the burden-sensing promoter is the promoter of the *mutM* gene. *mutM* encodes a functionally conserved DNA-glycosylase responsible for initiating repair of one of the most common oxidative stress induced DNA damage, i.e. oxidation of guanine to 7,8-dihydro-8-oxoguanine (8-oxoG) (Jain et al, 2007).

Other suitable burden-sensing promoters may be selected from among promoters of the *E. coli* genes *rrnB, rrnE, cspD, katE, xthA, uspE, gadB, ahpC, katG, grxA, oxyS, poxB, trxC.*

Burden-sensing promoters also include promoters related to a cell's carbon nutrient status and growth phase. Accordingly, in one embodiment, the burden-sensing promoter is the promoter of the toxin gene, *cspD*, which is activated by reduced growth rates and carbon starvation (Uppal et al., 2014; Yamanaka and Inouye, 1997) and hence predicted to be differentially up-regulated in cells subject to burden or fitness cost due to production of metabolites.

In one embodiment, the burden-sensing promoter is a ribosomal RNA promoter, in particular a promoter of *rrnB* and *rrnE* genes, that respond to the nutritional supply, are induced by the Fis protein, and are inhibited by ppGpp alarmone. *Rrn* promoter activity is induced in cells with high protein production, as seen in their early exponential growth phase (Nonaka et al, 2006).

In one embodiment, the burden-sensing promoter is one responsive to oxidative stress associated with metabolic burden and microbial heterologous production (Dragosits & Mattanovich, 2013). Oxidative stress response promoters include those promoters regulated by *E. coli* OxyR or its homologs such as *Bacillus* PerR, which activates several genes associated with amelioration and protection of the cell in response to oxidative damage; and promoters of genes belonging to rpoS (σ$^s$) that are upregulated during overexpression of proteins primarily during stationary growth phase.

For *Bacillus,* suitable burden-sensing promoters may be selected e.g. among HrcA regulated promoters including the promoter of *groES.* Other suitable burden-sensing promoters may be selected from among promoters of the B. subtilis genes *ctsR, dnaK, perR, hrc-4, spx, sigB, yflT, mutM.*

For Corynebacterium, such suitable burden-sensing promoters may be selected from among *katA, cpIX, mutM* and *groES.*

For Saccharomyes, suitable burden-sensing promoters may be selected from among promoter of genes *KAR2, PDI1, SAA1, FPR2, RPL3, RPL6A, RPL28, OGG1, RAD51, RAD54.*

For Aspergillus, such suitable burden-sensing promoters may be selected from among *bipA, PDI, clxA* and the homologues of *S. cerevisiae OGG1, RAD51 and RAD54.*

| Table 2 Non-exhaustive list of burden-sensing promoters | | | |
|---|---|---|---|
| No.* | Promoter | Encoded protein of gene regulated by the promoter | Ecocyc/ Biocyc accession ID**/ AspG D ID*** |
| 2 | p(*htpG*) (SEQ ID NO.: 65) | Heat shock protein G (HtpG), a member of the Hsp90 family, is a molecular chaperone that aids the refolding of protein aggregates in the cell. *htpG* transcription is activated via the $\sigma$32 regulon. (Yeast homolog: Hsp90) | EG10461-MONOMER |
| 3 | p(*ibpA*) (SEQ ID NO.: 66) | Small heat shock proteins LbpA and LbpB are chaperone proteins associated with heterologous protein expression and inclusion body formation that aids refolding of aggregated protein. *ibpA* transcription is activated via the $\sigma$32 regulon. | EG11534 |
| 4 | p(*clpB*) (SEQ ID NO.: 67) | ClpB is a caseinolytic protease catalyzing protein disaggregation. *clpB* transcription is activated via the $\sigma$32 and/or $\sigma$70 regulon. (Yeast homolog: Hsp104). | EG10157 |
| 5 | p(*yccV*) (SEQ ID NO.: 68) | YccV also known as HspQ participates in degradation of denatured protein during heat shock. *yccV* transcription is activated via the $\sigma$32 regulon. | G6500 |
| 6 | p(*grpE*) (SEQ ID NO.: 69) | GrpE is a nucleotide exchange factor in the DnaK-DnaJ-GrpE chaperone system. *grpE* promoter is activated via the $\sigma$32 regulon. | EG10416 |
| 7 | p(*ycjX*) (SEQ ID NO.: 70) | *ycjX* is activated via the $\sigma$32 regulon. | G6659 |
| 8 | p(*ldhA*) (SEQ ID NO.: 71) | D-lactate dehydrogenase and the *ldhA* promoter is activated via the $\sigma$32 regulon. | G592 |
| 9 | p(*mutM*) (SEQ ID NO.: 72) | DNA-formamidopyrimidine glycosylase is a component of the base excision repair pathway repairing certain types of DNA lesions. *mutM* promoter is activated via the $\sigma$32 regulon, but is inhibited by DksA-ppGpp binding under amino acid starvation. | EG10329 |
| 10 | p(*ybbN*) (SEQ ID NO.: 73) | YbbN also known as CnoX is a chaperedoxin. The *ybbN* promoter is activated via the $\sigma$32 regulon. | G6268 |
| 11 | p(*prlC*) (SEQ ID NO.: 74) | PrlC also known as oligopeptidase A; and belongs to the $\sigma$32 regulon. | EG11441 |
| 12 | p(*groES*) (SEQ ID NO.: 75) | GroES is a chaperonin, and belongs to the $\sigma$32 and $\sigma$70 regulon. | EG10600 |
| 13 | p(*fxsA*) (SEQ ID NO.: 76) | FxsA is a polytopic membrane protein belonging to the $\sigma$32 regulon. | G7832 |
| 14 | p(*htpX*) (SEQ ID NO.: 77) | HtpX is a zinc-dependent endoprotease and heat shock protein that belongs to the $\sigma$32 regulon. | EG10462 |
| 15 | p(*rrnB*) (SEQ ID NO.: 78) | 16S rRNA gene, whose promoter responds to nutritional supply, and is induced in cells with high protein production. | EG30085 |

(continued)

| No.* | Promoter | Encoded protein of gene regulated by the promoter | Ecocyc/ Biocyc accession ID**/ AspG D ID*** |
|---|---|---|---|
| Table 2 Non-exhaustive list of burden-sensing promoters | | | |
| 16 | p(rrnE) (SEQ ID NO.: 79) | 16S rRNA gene, whose promoter responds to nutritional supply, and is induced in cells with high protein production. | EG30088 |
| 19 | p(cspD) (SEQ ID NO.: 80) | CspD is a toxin. The cspD promoter responds inversely with growth rate. Transcription of cspD is activated by cyclic-AMP-CRP, H-NS and ppGpp. | EG11111 |
| 21 | p(katE) (SEQ ID NO.: 81) | Monofunctional catalase (HPII) responsible for decomposing the toxic hydrogen peroxide into water and oxygen in the cell. katE transcription is dependent on $\sigma^s$ and activated by Fur-Fe$^{2+}$ and ppGpp. | EG10509 |
| 22 | p(xthA) (SEQ ID NO.: 82) | Multifunctional endonuclease, Exodeoxyribonuclease III, that e.g. removes DNA damaged due to hydrogen peroxide. xthA belongs to the $\sigma^{32}$ regulon. | EG11073 |
| 23 | p(uspE) (SEQ ID NO.: 83) | Universal stress protein E; which is induced in stationary phase as a result of various stress conditions such as oxidative stress. uspE transcription is activated by GadX and ppGpp. | EG11246 |
| 24 | p(gadB) (SEQ ID NO.: 84) | Glutamate decarboxylase B helps the cell maintain a near-neutral intracellular pH upon exposure of acidic conditions by converting glutamate into gamma-aminobutyrate, accepting one proton in the reaction. gadB is a part of the the $\sigma^{70}$ and $\sigma^S$ regulon, and is activated by AdiY, GadE, GadX, P$^{asp56}$- RcsB and ppGpp. | EG11490 |
| 25 | p(ahpC) (SEQ ID NO.: 85) | Alkyl hydroperoxide reductase C plays a role in protecting cells from oxidative stress by catalyzing the reduction of endogenously synthesizes hydroperoxide. ahpC is part of the $\sigma^{70}$ and transcription is activated by OxyR. | EG11384 |
| 26 | p(katG) (SEQ ID NO.: 86) | Bifunctional hydroperoxidase I (HPI) that acts both as a catalase and a peroxidase. | EG10511 |
| 27 | p(grxA) (SEQ ID NO.: 87) | Glutaredoxin is mainly responsible for catalyzing the reduction of disulfides. Transcription initiation of grxA is activated by OxyR. | EG10417 |
| 28 | p(oxyS) (SEQ ID NO.: 88) | oxyS is a small RNA gene activated by oxidative stress via OxyR. | EG31116 |
| 29 | p(poxB) (SEQ ID NO.: 89) | Pyruvate oxidase that is upregulated by oxidative and osmotic stress via OxyR, but also by Cra, MarA, fnr, ppGpp and $\sigma^s$ | EG10754 |
| 30 | p(trxC) (SEQ ID NO.: 90) | Thioredoxin that is induces during oxidative stress via OxyR | EG11887 |
| *Saccharomyces* | | | |
| | p(KAR2) (SEQ ID NO.: 91) | HAC-upregulated promoter comprising an unfolded proteins response element, positively responding to high production | |

(continued)

| | | | |
|---|---|---|---|
| *Saccharomyces* | | | |
| | p(PDI1) (SEQ ID NO.: 92) | HAC-upregulated promoter comprising an unfolded proteins response element, positively responding to high production | |
| | p(SAA1) (SEQ ID NO.: 93) | HAC-upregulated promoter comprising an unfolded proteins response element, positively responding to high production | |
| | p(FPR2) (SEQ ID NO.: 94) | HAC-upregulated promoter comprising an unfolded proteins response element | |
| | p(RPL3) (SEQ ID NO.: 95) | RNA polymerase I upregulated promoter, positively responding to high production | |
| | p(RPL6A) (SEQ ID NO.: 96) | RNA polymerase I upregulated promoter, positively responding to high production | |
| | p(RPL28) (SEQ ID NO.: 97) | RNA polymerase I upregulated promoter, positively responding to high production | |
| | p(OGG1) (SEQ ID NO.: 98) | DNA damage response, resulting from heterologous expression broadly induces transcription of the DNA repair system. | |
| | P(RAD51) (SEQ ID NO.: 99) | DNA damage response, resulting from heterologous expression broadly induces transcription of the DNA repair system. | |
| | P(RAD54) (SEQ ID NO.: 100) | DNA damage response, resulting from heterologous expression broadly induces transcription of the DNA repair system. | |
| *Bacillus* | | | |
| | P*groES* (SEQ ID NO.: 212) | GroES is a chaperonin and is regulated by SigA and HrcA. | BSUB:BSU 06020-MONOMER |
| | P$_{ctsR}$ (SEQ ID NO.: 213) | CtsR is a transcription repressor of class III heat shock genes and is regulated by SigM, SigA, SigB, SigF, Spx and is also autoregulated. | BSUB:BSU 00830-MONOMER |
| | P$_{dnaK}$ (SEQ ID NO.: 214) | DnaK is a molecular chaperone and is regulated by HrcA and SigA | BSUB:BSU 25470-MONOMER |
| | P$_{perR}$ (SEQ ID NO.: 215) | PerR is a transcriptional repressor of the peroxide regulon and a sensor of the intracellular Fe/Mn ratio, and is regulated by SiqA and is also autoregulated. | BSUB:BSU 08730-MONOMER |
| | P$_{hrcA}$ (SEQ ID NO.: 216) | HrcA is a transcriptional repressor of class I heat-shock genes and is regulated by SigA and is also autoregulated | BSUB:BSU 25490-MONOMER |
| | P$_{spx}$ (SEQ ID NO.: 217) | Transcriptional regulator involved in the prevention of protein aggregation and is regulated by PerR, SigW, SigX, SigM, and SiqA | BSUB:BSU 11500-MONOMER |

(continued)

| Bacillus | | | |
|---|---|---|---|
| | P$_{sigB}$ (SEQ ID NO.: 218) | RNA polymerase sigma factor $\sigma^B$ involved in General stress response and is regulated by SigA and CcpA, and is also autorequlated | BSUB:BSU 04730-MONOMER |
| | P$_{yflT}$ (SEQ ID NO.: 219) | General stress protein 17M regulated by SigB | BSUB:BSU 07550-MONOMER |
| Asperqillus*** | | | |
| | pBipA (SEQ ID NO.: 189) | An ER localized chaperone (BiP) involved in protein secretion | AO090003 000257 |
| | pclxA (SEQ ID NO.: 190) | A calnexin homologue involved in protein secretion | AO090009 000313 |
| Corynebacterium ** | | | |
| | pgroES (SEQ ID NO.: 191) | Chaperone | G18NG-10159 |
| | pkatA (SEQ ID NO.: 192) | Catalase | G18NG-9810 |
| | pcplX (SEQ ID NO.: 193) | | |
| | pmutM (SEQ ID NO.: 194) | DNA damage repair | G18NG-11665 |
| * No. corresponds to the numbers assigned to the different strains tested in the Examples section of the present application.<br>** BioCyc/EcoCyc Database Collection is a large online collection of Pathway/Genome Databases - accessed through https://biocyc.org/<br>*** For Aspergillus, Aspergillusgenome.org is a database (AspGD) featuring the genomes of relevant Aspergillus species. | | | |

The basal expression level and specific response curve upon induction/activation of the different burden-sensing promoters (e.g. in Table 2) vary, which can be exploited when selecting a burden-sensing promoter having the best match (in terms of strength) to a specific microbial production cell and its product.

In some cases, the gene of interest is part of an operon, in which case the first gene of the operon is preferably used to define the relevant promoter sequence.

Suitable burden-sensing promoters that are induced by the burden and/or fitness cost in a microbial production cell during production can be selected from public databases known to the skilled person, for example https://ecocyc.org for E. coli promoters; https://bsubcyc.org for Bacillus promoters (which also works for Corynebacterium promoters), their respective prokaryotic homologues; https://yeastgenome.org for Saccharomyces cerevisiae promoters, and their fungal homologues; and https://aspergillusgenome.org is a database (AspGD) featuring the genomes of relevant Aspergillus species.

Suitable burden-sensing promoters can also be validated in a test assay in which a candidate burden-sensing promoter is operably linked to a gene encoding a fluorescent protein (e.g. green fluorescent protein) and integrated into a microbial production cell genetically engineered to synthesize a product, and integrated into a corresponding non-producing cell (control), which was isolated following a serial dilution cultivation experiment in production medium for between 50-150 cell generations or from the end of a large scale fermentation culture. Burden-sensing promoters induce transcription of the candidate burden-sensing promoter operably linked to fluorescent protein expression in producing cells compared to the non-producing cell (control) by at least 5 %, 7.5 %, 10 %, 15 %, 25 %, 60 %, 150 %, 300 % in the production cell compared to the activity in the isolated corresponding non-producing cell.

*II.iv Matching a burden-sensing promoter to a microbial production cell*

Microbial production cells, in response to the burden or fitness cost of producing a product, exhibit a transcriptional state characterized by the expression of certain genes, whose expression is not induced in a corresponding non-productive parent cell or a non-productive escape cell derived from a microbial production cell during production fermentation. The physiological nature of the burden or fitness cost will depend on the product synthesized by a given microbial production cell, and will be reflected in the types of genes whose expression is induced. The identity of the gene promoters induced in a cell producing a given product can be determined by techniques well-known in the art (e.g. transcriptomics, see example 7). Since many of the induced gene promoters will be related to, or correspond to those listed in Table 2, these provide a starting point for finding a matching burden-sensing promoter for a given microbial production cell. The choice of promoters can be extended by identifying 5 to 15 promoters of genes found to be specifically upregulated in the production strain of interest. As illustrated in the examples herein, the method used to match a burden-sensing promoter operably linked to an essential gene to a microbial production cell in a product-specific manner, is experimentally fast and can be conducted in simple laboratory setups.

*II. v Translation control elements*

Regulating the growth of a microbial production cell of the invention by means of essential gene expression, requires that the response threshold and curve of the burden-sensing promoter and the expression level of the essential gene are balanced; such that basal level essential gene expression supports limited or no growth, while in highly productive cells the induced burden-sensing promoter drives sufficient essential gene expression to support a significantly increased growth rate, preferably a growth rate similar to that of productive cells lacking the burden-sensing promoter of the invention or ≤5 % lower, when measured in the exponential growth phase.

One suitable approach to balancing the burden-sensing promoter's burden-response to the expression level of the essential gene is to modify the translational strength of the essential gene. In bacteria, translational strength is defined by the Shine-Dalgarno/ribosome binding site (RBS) sequence directly upstream of the start codon, while in eukaryotic cells translation initiation regions/Kozak elements can be used to modify translational strength. RBSs in *E. coli* conferring a broad range of translational strengths are provided in Table 3.1, while further examples can be found in the literature (e.g. Bonde et al, 2016). A skilled person in the art can balance the translational strength of the regulated essential gene by constructing four variants of the ribosomal binding site for each burden-sensing promoter (Rugbjerg et al, 2018, PNAS), and testing which variant enables a selected essential gene to effectively regulate the growth rate of a cell. Exemplary RBSs for use in *Bacillus licheniformis* or *Bacillus subtilis* are provided in Table 3.2.

**Table 3.1** List of suitable RBSs in *E. coli* generally covering a broad expression range as defined in assay by expression of red fluorescent protein (Bonde et al, 2016).

| RBS | Log (Expression level) |
| --- | --- |
| ACTTGA | 2.80244 |
| ACTTGC | 2.73094 |
| ACTTGG | 3.14847 |
| ACTTGT | 2.77523 |

**Table 3.2** List of suitable RBSs in *B. subtilis* or *B. licheniformis* generally covering a broad expression range.

| RBS |
| --- |
| ACGAGA |
| ACGAGC |
| ACGAGT |
| ACGAGA |

*II.vi Engineering burden-sensing promoters and translational control element*

A native burden-sensing promoter of choice is generally encompassed by the -1 to -300 bp region (upstream) of the native regulated ORF in prokaryotic organisms and the -1 to -500 bp region in eukaryotic organisms. Core promoters that must be included and the sequence boundaries of their regulatory sites e.g. transcription factor binding sites, are common general knowledge, such as for $\sigma32$ (Nonaka et al, 2006). The translational control element/RBS can be added downstream of the promoter, to avoid alteration in the regulatory properties of the selected promoter sequence.

*II.vii Improved production levels*

A surprising advantage of the microbial production cell of the invention, in addition to having an increased initial growth rate as compared to non-productive cells, is that the cells retain significantly improved productivity during a large scale fermentation (simulated in example 2; figure 6), over many cell divisions, compared to microbial production cells lacking engineered burden-addiction. The surprising advantages conferred by burden-addiction in a microbial production cell of the invention are obtained irrespective of the type of product synthesized - as illustrated by the range of products synthesizes by microbial production cells in examples 2-5.

According to one embodiment, the microbial production cell of the invention is characterized by improved product yield following at least 20, 25, 30, 35, 40, 45, 50, 55, 60, 70, 100, 150, 250 or 400 generations of cell division from a single cell, compared to a non-burden-addicted production cell following the same generations of cell division. Product yield is measured as moles or grams of product produced per unit substrate.

In one embodiment, production levels are increased by at least 10, 25, 50, or 80% following at least 50 generations of cell division from single cell, compared to a reference non-burden-addicted production cell following the same generations of cell division.

Universal practice in the engineering of microbial cell factories has aimed at minimizing the burden known to arise due to maintenance and expression of heterologous pathways. The present invention provides a counter-intuitive solution to improving cell factory yields, since it makes microbial cell growth and survival dependent on the cell producing its product while being subject to a constant burden or fitness cost. While not wishing to be bound by theory, it is speculated that the selection pressure placed on a microbial production cell of the invention and its descendants, where only productive cells in a state of burden can grow/survive, may serve to progressively select for highly-burdened cell sub-populations during long-term culture in a starting population of initially isogenic cells.

## III. Methods for preparing and identifying cells of a burden-addicted microbial production strain

In a preferred method of performing the invention, a burden-addicted microbial production strain is prepared and identified by the following steps. To obtain the best working relation between burden sensing promoter and essential gene, it may be relevant to prepare and test different candidate burden-sensing promoters with different RBSs for regulating a selected essential gene.

*III.i Identification of burden-sensing promoter candidate(s) in a specific production strain*

Candidate burden-sensing promoters listed in Table 2 may be tested. Alternatively, unique, positively differentiating gene transcripts detected in a specific production strain of interest may be identified; whose respective promoters provide a source of candidate burden sensing promoters. Preferably such differentiating gene transcripts are identified by comparing the transcript profile of a microbial production strain (during production) with an isolated genetic or non-genetic escape mutant variant(s) derived from the corresponding production strain, where such escape mutant variant is characterized by at most a 50 % lower production rate. Positively differentiating gene transcripts can be identified by use of transcriptomics; for example by RNA sequencing of the transcribed RNA extracted from productive microbial production cell(s) compared to the non/low-producing escape mutant variant. Identified promoters can be tested in combination with a given essential gene in a microbial production cell.

A method for identifying at least one preferably two burden-sensing promoters is illustrated in example 7; where after said promoter(s) is operably linked to an essential gene in a chosen microbial production cell for testing.

*III.ii Introduction of burden-sensing promoter in growth-regulating processes*

The at least one preferably two promoter(s) selected from the list of general candidate burden-sensing promoters (Table 2) or from identified specific burden-sensing promoter(s) (see IIIi), are tested by operably linking it to an essential gene in the microbial production cell. Different translation strengths of the essential gene can be simultaneously tested by providing alternative RBS sequences for the cognate essential gene; as well as testing different essential genes (as describe in section Iiii). The essential gene may be native or heterologous with respect to the microbial production cell.

When the essential gene is a native gene, its cognate native promoter may be disrupted (i.e. made non-functional) by e.g. mutation or deletion events, and the burden-sensing promoter (that is heterologous with respect to the essential gene) is then operably linked to the native essential gene by targeted introduction. In another embodiment, the native essential gene promoter is replaced with the burden-sensing promoter by targeted introduction.

Suitable methods for targeted introduction of a genetic sequence in microorganisms include recombineering in bacteria, e.g. Lambda Red recombineering in *E. coli*; while homologous recombination can be used in yeasts and filamentous fungi. Both concepts can optionally be used in combination with CRISPR to increase gene replacement efficiency.

*III.iii Screening for balanced burden-sensing and growth-regulation*

Microbial production cell clones comprising an inserted burden-sensing promoter operatively linked to an essential gene are then screened to identify clones where the burden-sensing promoter regulates an essential gene in a growth-controlling manner. For example, the growth of a number of such clones (e.g. 8-96 clones) is compared with cells of a corresponding non-burden-addicted microbial production strain, under conditions were product production is controlled (e.g. using microbial production strains where the production gene is inducible).

When cell growth is measured under conditions where product synthesis by both strains is low/absent; a suitable burden-addicted production clone is one that exhibits a significant, and preferably at least 5 %, lower growth rate than the non-burden-addicted production strain (Example 1, Figure 2). If instead production is high/present; the up-regulation of essential gene expression in a suitable burden-addicted production clone, means that the difference in growth rate is significantly reduced or eliminated when compared to the corresponding non-burden addicted production strain.

*III.iv Validation of non-perturbed central and production metabolism*

Changes in transcriptional regulation or expression of essential genes can lead to unwanted, indirect perturbation of the production genes and central carbon or nitrogen metabolism, compromising product formation and in turn reduced burden in a burden-addicted microbial production cell. In order to exclude such clones, cell growth is measured under conditions where both strains synthesize product; where a suitable burden-addicted production clone is one that exhibits a growth rate equal to or lower than the non-burden-addicted production strain (as seen in example 2, figure 7).

*III.v Screening for growth-rate stability*

Clones that fulfil the above criteria are tested for growth rate stability under production-mimicking conditions by cultivation for at least 20 generations of cell division, e.g. by serial passage and measurement of growth rates, or by taking samples from various steps in a scaled-up production process. Suitable burden-addicted microbial production cells maintain growth rates lower than the non-burden-addicted production strain over time (e.g. example 2, figure 7).

**IV. Method for producing a desired product using cells of a burden-addicted microbial production strain**

A second aspect of the present invention concerns a method for producing a desired product comprising the steps of:

a. providing at least one microbial production cell genetically engineered to synthesize a product, wherein said at least one cell further comprises at least one preferably two essential gene(s) operably linked to at least one preferably two burden-sensing promoter(s), respectively, and wherein said promoter(s) are heterologous with respect to said essential gene,

b. introducing the at least one genetically modified microbial cell into a cultivation medium comprising substrate for production of said product,

c. recovering said product synthesizes by said culture,

wherein synthesis of the product confers a burden and/or fitness cost on said at least one cell, and wherein expression of said at least one preferably two essential gene(s) are up-regulated relative to basal level expression of said at least one preferably two essential gene(s) when said at least one preferably two burden-sensing promoter(s) are induced by said burden and/or fitness cost; and wherein a lack of said product synthesis in said at least one production cell or progeny cell thereof reduces growth rate of said at least one cell.

The method for producing the desired product may comprise the step of providing a cell culture of said at least one microbial production cell genetically engineered to synthesize a product; and introducing the cell culture of the at least one genetically modified microbial cell into a cultivation medium comprising substrate for production of said product.

## V. Use of cells of a burden-addicted microbial production strain for producing a desired product

A third aspect of the present invention concerns the use of a burden-addicted microbial production cell of the invention for producing a desired product, wherein a lack of product synthesis in said a burden-addicted production strain or progeny cell thereof reduces growth rate of said strain even if product is present in the intracellular or extracellular environment.

## EXAMPLES

### Example 1: Engineering burden-addicted E. coli strains producing recombinant human growth hormone

*1.1 Maintenance and expression of heterologous production genes by a cell factory constitutes an unnatural burden on its cells*

The growth rate of parent *E. coli* BL21(DE3) cells was compared with an engineered derivative transformed with the plasmid, pEG34, comprising a gene encoding recombinant human growth hormone (hGH) fused to Green Fluorescent Protein (GFP).

1.1.1 Materials and Methods

Cells of the host strain *E. coli* BL21(DE3) (E. *coli* Genetic Stock Center at Yale University), were made electrocompetent and transformed by standard electroporation methods (1800 V, 25 $\mu$F, 200 Ohms, 1 mm cuvette width) with the plasmid pEG34, and plated on LB agar plates containing chloramphenicol.

| Table 4 Plasmids | | |
|---|---|---|
| Plasmid | Genotype | Expressed proteins |
| pEG34 [SEQ ID No.: 101] | hGH-GFP fusion camR gene* [SEQ ID No.: 103] | hGH fused to GFP [SEQ ID No.: 102] Chloramphenicol resistance protein [SEQ ID No.: 104] |
| pEG0 [SEQ ID No.: 105] | *camR* gene* [SEQ ID No.: 103] | Chloramphenicol resistance protein [SEQ ID No.: 104] |
| pENDU5CAM [SEQ ID No.: 106] | *lss* gene *camR* gene* [SEQ ID No.: 103] | Lysostaphin [SEQ ID No.: 107] Chloramphenicol resistance protein [SEQ ID No.: 104] |
| pMevT [SEQ ID No.: 108] | *mevT* operon *camR* gene* [SEQ ID No.: 103] | Acetoacetyl-CoA synthase [SEQ ID No.: 109] Hydroxymethylglutaryl-CoA synthase [SEQ ID No.: 110] Hydroxymethylglutaryl-CoA reductase [SEQ ID No.:111] Chloramphenicol resistance protein [SEQ ID No.: 104] |
| pSIM5-tet [Koskiniemi et al 2011] | | |
| *camR gene [SEQ ID No: 103] encoding chloramphenicol resistance protein [SEQ ID No: 104] is in pEG34 [SEQ ID No: 101] located at nucleotide positions 3768-4427 on the complementary strand; is in pEG0 [SEQ ID No: 105] located at nucleotide positions 2339-2995 on the complementary strand; is in pENDU5CAM [SEQ ID No: 106] located at nucleotide positions 1617-2276 on the complementary strand; and is in pMevT [SEQ ID No: 108] located at nucleotide positions 6585-7244 on the complementary strand. | | |

Pre-cultures of single colony transformants were cultured in 96-well microtiter plates on 200 $\mu$L 2xYT medium (16 g/L tryptone, 5 g/L NaCl, 10 g/L yeast extract) containing 500 $\mu$M IPTG and 30 mg/L chloramphenicol at 37 °C with fast horizontal shaking in an Elx808 plate reader (Biotek) with reads at OD630 every ten minutes. OD630 values were background-subtracted using the OD630 value of the first read.

1.1.2 Results

*E. coli* cells comprising the hGH-GFP expression plasmid, pEG34, grow slower than cells of the parent *E. coli* host from which is was derived (figure 1); illustrating the burden or load placed on cells as a result of allocating cellular resources to maintenance and expression of a synthetic construct.

*1.2 Engineering a burden-addicted cell factory*

Burden-addiction strains of the parent *E. coli* host were genetically engineered to incorporate a genetic circuit designed to confer a selective fitness advantage on productive cells in a cell factory. Specifically, a burden-sensing promoter ($p_{mutM}$, $p_{yccV}$ or $p_{ycjX}$), was substituted for the native promoter of the essential gene operon *folP-glmM* in the *E. coli* BL21(DE3) chromosome. The genetic circuit further included an RBS, between the promoter and essential gene, where four different RBSs (Table 3) were tested for modulating the expression level of the essential gene. The growth rate of strains comprising the genetic circuit was compared with the parent host strain *E. coli* BL21(DE3).

1.2.1 Materials and Methods

*Burden-sensing promoters:* The promoters, $p_{mutM}$, $p_{yccV}$ or $p_{ycjX}$, were generated by PCR amplifying a 0.3 kb region immediately upstream of the respective gene (see Table 3) using primers specified in Table 5 and genomic DNA derived from lysed *E. coli* BL21(DE3) cells as template. The PCR mix: 10 $\mu$l MQ water, 2 $\mu$l forward primer (10$\mu$M), 2 $\mu$l revers primer (10$\mu$M), 1$\mu$l DNA template, 15 $\mu$l Phusion U MasterMix (Thermo Scientific). The PCR reaction protocol: 95°C for 180 sec (1x); 95 °C for 20 sec, 68-58°C (touchdown) for 30 sec, 72°C for 60 sec (35x); 72°C for 300 sec (1x); leave at 15°C.

**Table 5 Primers**

| Promoter of *E. coli* gene | Primer name | Primer sequence (5'-3')* | SEQ ID No.: |
|---|---|---|---|
| yccV | yccV for | ATTCATATGUGGTTATAAAGACATCAACATGC | 112 |
| | yccV rev | ATCCCCATTACGTGAGGATGGCTAAGGTCCAGTGAAGTACCCTGGGCAAAGAGTTTCATacctt**N****CAAGT**AGTCACCTCCCGGGAAATCT | 113 |
| ycjX | ycjX for | ATTCATATGUTTGGCTGATACTGTAATTCTTC | 114 |
| | ycjX rev | ATCCCCATTACGTGAGGATGGCTAAGGTCCAGTGAAGTACCCTGGGCAAAGAGTTTCATacctt**N****CAAGT**TGCTGTCCTGTGCTGCTCTG | 115 |
| mutM | mutM for | ATTCATATGUGGCGAAAAGTACTAAGTACTTA | 116 |
| | mutM rev | ATCCCCATTACGTGAGGATGGCTAAGGTCCAGTGAAGTACCCTGGGCAAAGAGTTTCATacctt**N****CAAGT**AGCATCTCCAGGAATGAACA | 117 |
| | P492 | TGCAACTCTTCTTCAACGC | 118 |
| | P493 | AAGAACCAGGCGCTTCTAA | 119 |
| | P525 | ATTGATGACCTGATGGCAC | 120 |
| | P526 | GATCCAGACTTCGAAGCG | 121 |
| * RBS complementary coding sequences are in bold font; wherein N is either A, G, T or C. The RBS is encoded on the opposite strand of the oligo sequence shown. | | | |

USER cloning was used to generate integration sequences comprising the amplified promoter region fused to a linear 1.5 kb DNA fragment containing a *kanR* gene for selection of correct recombineering products and a 221 bp targeting sequence identical to 221 bp directly upstream of the *folP* gene. The amplified promoter region and kanR-folP fragment were fused by mixing them in equimolar amounts and adding 1μl 10x T4 ligation buffer (Thermo Scientific) and 0.75 μl USER enzyme (New England Biolabs), in a total 10μl reaction volume. The USER reaction was placed at 37°C for 30 minutes. The reaction was then placed at room temperature for 15 minutes followed by the addition of 0.75 μl T4 DNA ligase (Thermo Scientific) and incubation at room temperature for 30 minutes. An example of such integration sequence comprising promoter, KanR resistance gene, and folP targeting sequence can be found in the sequence listing SEQ ID NO. 140 (s9_pmutM_folP). The ligated product was then amplified using primers'rev' (according to specific promoter in Table 5) and P493 (Table 5) to approximately 250 ng/μl using the PCR reaction protocol: 98°C for 180 sec (1x); 85°C for 20 sec, 72-68°C (touchdown) for 30 sec, 72°C for 60 sec (35x); 72°C for 5 min (1x); leave at 15°C. Primer overhang provided 50 bp *folP* identical targeting sequence to direct recombineering to the *folP* locus.

*Chromosomal integration of burden-addiction promoters:* promoters were integrated upstream of the *folP* gene in the genome of *E. coli* BL21(DE3) cells as follows: 100 ml of 2xYT medium containing tetracycline were inoculated with 600μl of BL21(DE3) overnight culture pre-transformed with pSIM5-tet (Koskiniemi et al, 2011). The cells were cultured at 30°C, and upon reaching OD600=0.20, the culture was transferred to a 42°C shaking bath for 15 minutes to allow for the expression of the recombineering enzymes located on pSIM5-tet. The culture was then transferred to 2x cold 50ml centrifuge tubes; centrifuged at 4000g for 10 min; the supernatant was discarded; and the remaining cell pellets were washed with 20 ml ice-cold 10% glycerol. The partially re-suspended cells were then centrifuged at 4000g for 6 min; the supernatant was discarded; and the cell pellets washed with 20 ml ice-cold 10% glycerol. The partially re-suspended cells were centrifuged at 4000g for 6 min; each cell pellet was carefully re-suspended in 495 μl ice-cold 10% glycerol and pooled. 90 μl of re-suspended cells were added to electroporation cuvettes each containing 1 μl (>250 ng) of one of the burden-sensing promoter integration sequences. The cells were electroporated with the following settings: 1800 V, 25 μF, 200 Ohms, 1 mm cuvette width. 900 ml 2xYT-media was added to the cuvette straight after electroporation and the cells were left to recover for 1.5 hour at 37°C in 1.5 ml Eppendorf tubes. The electroporated cells were incubated at room temperature overnight to allow time for recombination; and subsequently plated on LB agar plates containing 50 mg/L kanamycin and cultured at 37°C overnight to ensure curing for pSIM5-tet. Correct targeting to the essential gene locus was validated using primers P525 and P526. Generally, single kan[R] colonies were picked having a size average or smaller than the population of colonies.

Burden-addiction promoter integration in the genome of the kanR selected colonies was validated by colony PCR using Taq DNA polymerase and primers targeted to the *folP* promoter region. Additionally, the identity of the RBS in the selected colonies was determined by Sanger sequencing.

Subsequently, the growth rate of strains comprising the genetic circuit was compared with the parent host strain *E. coli* BL21(DE3) by cultivation in 200μl 2xYT supplemented with 500 μM IPTG at 37 °C with horizontal shaking.

1.2.2 Results

The growth rate of the selected burden-addicted *E. coli* strains were slower, to various degrees, compared to parent *E. coli* host from which they were derived (figure 2; showing growth of burden-addicted strains s5.0#3, s7.0#8, and s9.0#8 wherein essential genes *folP-glmM* are controlled by burden-sensing promoters $p_{ycc}v$, $p_{ycj}x$, and $p_{mutM}$, respectively). Since cellular growth is dependent on the level of expression of the essential *folP-glmM* genes, it may be concluded that the level of essential gene expression driven by the respective burden-sensing promoter in a non-productive, burden-addicted strain is insufficient to support growth at the level of a wildtype parent strain.

The observed reduction in growth rate in these burden-addicted strains provides a measure of the penalty that may be exerted on a non-producing cell that evolves spontaneously during cultivation of a cell factory population comprising the burden-addiction genetic circuit. The degree of penalty is determined by the choice of the burden-sensing promoter combined with the strength of the chosen RBS. The larger the penalty - the wider the window for "negative selection" of any low- or non-producing variants (e.g. resulting from mutation in production genes) spontaneously arising during cultivation.

*1.3 Production increases growth rate of a burden-addicted cell factory*

The burden-addicted *E. coli* strains, which conferred a growth penalty for non-production (figure 2), were used as host strains for demonstrating the selective fitness advantage for burden-addicted cells that are productive.

1.3.1 Materials and methods

The slowest-growing clones of the generated burden-addicted *E. coli* strains (s9.0#8; s5.0#3; and s7.0#8) were made electrocompetent and transformed by standard electroporation methods (1800 V, 25 $\mu$F, 200 Ohms, 1 mm cuvette width) with respectively pEG34 or pEG0 (Table 4), and plated on LB agar plates containing chloramphenicol and kanamycin. Single colony transformants were cultured over-night on 2xYT medium containing chloramphenicol, and then used to inoculate 96-well microtiter plates comprising 200 $\mu$L 2xYT medium (500 $\mu$M IPTG, chloramphenicol) and cultured at 37 °C with fast horizontal shaking in an Elx808 plate reader (Biotek) with reads at OD630 every ten minutes. OD630 values were background-subtracted using the OD630 value of the first read.

*E. coli* strains were each transformed with the plasmid pEG34 or pEG0 (Table 4), and their growth rate properties were measured when the cells were induced to synthesize the recombinant protein, hGH fused to GFP.

1.3.2 Results

The burden-addicted strain, *E. coli* strain s9.0#8, comprising the burden sensing promoter $p_{mutM}$ controlling of the *folP-glmM* essential genes expression, exhibited the slowest growth of the strains tested when compared to the parent *E. coli* BL21 (DE3) strain (figure 2). This reduction of exponential growth rate is reversed in cells of the *E. coli* strain s9.0#8 transformed with the pEG34 plasmid and induced to express the recombinant protein, hGH-GFP, in contrast to cells of *E. coli* strain s9.0#8 transformed with the empty plasmid, BEG0 (figure 3). This demonstrates that the burden-sensing promoter $p_{mutM}$ in *E. coli* strain s9.0#8 is induced by the burden or load on the cells brought about by synthesis of the recombinant protein hGH-GFP. This, in turn, leads to up-regulated expression of the *folP-glmM* genes and enhanced exponential growth.

The synthesis of hGH-GFP in each of the burden-addicted strains, *E. coli* strain s5.0#3, s7.0#8, and s9.0#8 harboring the pEG34 plasmid, not only led to a reversal of the reduction of exponential growth rate; but additionally the growth rate of these strains was indistinguishable from growth of the parent *E. coli* BL21 (DE3) (figure 4).

*In summary,* this example illustrates that the burden-addiction genetic circuit can be used to confer a selective fitness advantage on those cells of a cell factory whose synthesis of proteins or metabolites is sufficiently high to constitute a burden or load; and that this burden or load is detectable by a burden-sensing promoter operably linked to an essential gene in the cells. By contrast, non-productive variant cells (e.g. resulting from production gene mutations), that spontaneously appear during cultivation of the burden-addicted cell factory, will be subject to a negative selection pressure, since their growth will be slowed to a rate supported by the basal expression of the essential gene. A reduced growth rate is, of itself, sufficient to slow the increase in frequency of such variant cells in the cell factory and thereby delay the decline in cell factory productivity over time.

**Example 2: Burden addicted *E. coli* strains producing human growth hormone show enhanced long-term production stability**

Burden-sensing promoters are shown to be promoters that can sense and be activated by a burden-induced state in a cell resulting from the cells synthesis of a recombinant protein, hGH-GFP. Once activated, burden-sensing promoters are shown to elevate expression of an essential gene, *folP-glmM,* to a level sufficient to confer a selective growth advantage on a cell when compared to a non-productive cell. In order to maximize the dynamic range of essential gene expression in response to its cognate burden sensing promoter, the burden-sensing promoter is randomly combined with variant RBS coding sequences (Table 3) conferring different translational strengths. Promoters having burden-sensing properties suitable for use in a burden-addiction genetic circuit are shown to include heat-shock-, DNA damage-, oxidative stress response-promoters and rRNA promoters, as illustrated by the following engineered production strains cultured under simulated large-scale production conditions.

Firstly, a number of clones harboring random variant RBS coding sequences were selected for each of the burden-sensing promoters, and their hGH-GFP synthesis was followed over many cell divisions in order to demonstrate their relative ability to elevate/preserve recombinant hGH-GFP synthesis over time.

*2.1 Materials and Methods*

*Burden-sensing promoters:* The promoters, $p_{yccV}$, $p_{ycjX}$, $P_{ibpA}$, $p_{grpE}$, $P_{ldhA}$ and $p_{ybbN}$, and their respective integration sequences were generated by PCR and USER cloning as described for Example 1.2.1, using the primers specified in Table 6; while the ligated products were amplified using primers'rev' (according to specific promoter in Table 6) and P493 (Table 5).

| Table 6 Primers | | | |
|---|---|---|---|
| Promoter of *E. coli* gene | Primer name | Primer sequence (5'-3')* | SEQ ID No.: |
| ibpA | ibpA fwd | ATCCCCATTACGTGAGGATGGCTAAGGTCCAGT GAAGTACCCTGGGCAAAGAGTTTCATacctt**NCA AGT**AATCAATAGCTCCTGAAATC | 122 |
| | ibpA rev | ATTCATATGTTTGGCCTGATGAGTTATAGCG | 123 |
| grpE | grpE fwd | ATTCATATGUTCTCCGCGAGCGTGCCAGTTTT | 124 |
| | grpE rev | ATCCCCATTACGTGAGGATGGCTAAGGTCCAGT GAAGTACCCTGGGCAAAGAGTTTCATacctt**NCA AGT**GAATTTCTCCGCGTTTTTTT | 125 |
| ldhA | idhAfwd | ATTCATATGUGGGAACCCACAGCCCGAGCGTC | 126 |
| | ldhA rev | ATCCCCATTACGTGAGGATGGCTAAGGTCCAGT GAAGTACCCTGGGCAAAGAGTTTCATacctt**NCA AGT**AAGACTTTCTCCAGTGATGT | 127 |
| ybbN | ybbN fwd | ATTCATATGUCCCATTCGTACTCGCTTCACCGAT CCCCATTACGTGAGGATGGCTAAGGTCCAGTGA AGTACCCTGGGCAAAGAGTTTCATacctt**NCAAG T**GGAGTCGCTCTCTGTTGTCG | 128 |
| | ybbN rev | ATCCCCATTACGTGAGGATGGCTAAGGTCCAGT GAAGTACCCTGGGCAAAGAGTTTCATacctt**NCA AGT**GGAGTCGCTCTCTGTTGTCG | 129 |
| fxsA | fxsA fwd | ATTCATATG UTAAACACACAGAATATATGTGG | 130 |
| | fxsA rev | ATCCCCATTACGTGAGGATGGCTAAGGTCCAGT GAAGTACCCTGGGCAAAGAGTTTCATacctt**NCA AGT**AGGTTTCTCCTGTAATAGCA | 131 |
| rrnB | rrnB fwd | ATTCATATGUGCAAACACGCCGCCGGGTCAG | 132 |
| | rrnB rev | ATCCCCATTACGTGAGGATGGCTAAGGTCCAGT GAAGTACCCTGGGCAAAGAGTTTCATacctt**NCA AGT**AAAAGTTTGACGCTCAAAGA | 133 |
| rrnE | rrnE fwd | ATTCATATGUAATGCGCCTCCGTTGAGACGACA AC | 134 |
| | rrnE rev | ATCCCCATTACGTGAGGATGGCTAAGGTCCAGT GAAGTACCCTGGGCAAAGAGTTTCATacctt**NCA AGT**AAAAGTTTGATGCTCAAAGA | 135 |

(continued)

| Table 6 Primers | | | |
|---|---|---|---|
| Promoter of *E. coli* gene | Primer name | Primer sequence (5'-3')* | SEQ ID No.: |
| poxB | poxB fwd | ATTCATATGUTAGGTTGTCGCTGCCTGCCGTG | 136 |
| | poxB rev | ATCCCCATTACGTGAGGATGGCTAAGGTCCAGT GAAGTACCCTGGGCAAAGAGTTTCATacctt**NCA AGT**GGTTCTCCATCTCCTGAATG | 137 |
| * RBS complementary coding sequences are in bold font; wherein N is either A, G, T or C. The RBS is encoded on the opposite strand of the oligo sequence shown. | | | |

*Chromosomal integration of burden-addiction promoters:* each of the promoters was integrated upstream of the *folP* gene in the genome of *E. coli* strain BEG34 (corresponding to *E. coli* BL21(DE3) harboring plasmid pEG34), as follows. Cells of the *E. coli* strain BEG34, pre-transformed with the recombineering plasmid pSIM5-tet, were prepared and transformed by electroporation with each of the promoter integration sequences, as described in example 1.2.1. Following the described steps of recombineering and curing of pSIM5-tet, eight colonies were picked from each plate, corresponding to eight clones with the same promoter integration but having a random variant RBS sequence (see Table 6). Each clone was then transferred to a well of a 96-well plate containing 200 μl 2xYT supplemented with chloramphenicol for maintenance of the plasmid, pEG34. 2 μl of each cultured clone was used to validate promoter integration via colony PCR as described in example 1.2.1; prior to freezing the 96-well plate.

*Short term hGH-GFP production screening assay*: The frozen 96-well plate was thawed and a pin replicator used to transfer cells into a new 96-well plate containing 200 μL 2xYT supplemented with chloramphenicol. This plate was sealed with Breathe-Easy sealing membrane (Sigma-Aldrich) and placed in a SynergyH1 plate reader (Biotek) overnight at 37°C and 754 rpm linear shaking for 20 hours; and the OD (600nm) and GFP fluorescence (ex/em 485nm/528nm) of each well was measured every 10 minutes over a 20 hour period. The plate was then placed in a regular tabletop plate shaker at room temperature for 4 hours followed by the transfer of 2 μL culture from each well to a new 96-well plate containing 200 μL 2xYT supplemented with chloramphenicol and 0.5mM IPTG. The new 96-well plate was likewise sealed with Breathe-Easy sealing membrane (Sigma-Aldrich) and placed in a SyngergyH1 plate reader overnight at 37°C and 754 rpm linear shaking for 20 hours; and the OD and GFP fluorescence of each well was measured at 600nm and ex/em 485nm/528nm every 10 minutes for 20 hours. The following day 2 μL culture was transferred to a new 96-well plate with 200 μl 2xYT supplemented with chloramphenicol and 0.5mM IPTG and the process was repeated every day for 6 days in total.

*Long term hGH-GFP production assay (figure 6):* A single selected colony from each burden-addicted hGH-GFP producing strain was used to inoculate a 15 mL Greiner culture tube containing 4 mL 2xYT supplemented with chloramphenicol and 0.5mM IPTG. The cultures were grown for 23 hours at 37°C on a 250 rpm shaking table. 2 μL of each culture were then seeded in 2000x dilution (corresponding to approx. 11 generations of cell division) into a new Greiner culture tube under identical conditions, where these method steps were repeated for a total of 12 seeds. After each passage, 200 uL samples of the culture were taken to determine hGH-GFP synthesis by measuring OD600 and GFP fluorescence (ex/em 485nm/528nm) in a SynergyH1 plate reader (Biotek).

*Long term hGH-GFP production assay (figure 8):* A single selected colony from each burden-addicted hGH-GFP producing strain was used to inoculate a 24 deep-well plate carrying 1.8 mL 2xYT supplemented with chloramphenicol and 0.5mM IPTG. The cultures were grown for 23 hours at 30°C on a 200 rpm shaking table. 2 μL of each culture were then seeded in 1000x dilution (corresponding to approx. 10 generations of cell division) into a new deep well plate under identical conditions, where these method steps were repeated for a total of 9 seeds. After each passage, 200 uL samples of the culture were taken to determine hGH-GFP synthesis by measuring OD600 and GFP fluorescence (ex/em 485nm/528nm) in a SynergyH1 plate reader (Biotek).

*Growth rate measurements of chosen strains*: Selected high-hGH producing strains, s3.6#2, s6.6#6, s7.6#8 and s10.6#7, were streaked on LB agar plates containing chloramphenicol and kanamycin. BEG34 and BEG0 strains were streaked on LB agar plates containing chloramphenicol. 7 colonies from each plate were used to inoculate a 96-well plate containing 200 μl 2xYT supplemented with chloramphenicol; which were sealed with Breathe-Easy sealing membrane (Sigma-Aldrich). Their growth was measured in a SynergyH1 plate reader (Biotek) overnight at 37°C and 754 rpm linear shaking. 2 μl samples from each well were transferred to wells in a new 96-well plate containing 200 μl 2xYT supplemented with chloramphenicol and 0.5mM IPTG. The 96-well plate was sealed with breathe-easy film and incubated for 20 hours at 37°C and 754 rpm linear shaking and growth (OD600nm) was measured every 10 minutes using a Synergy H1 plate reader.

*Strain catalogue:*

BEG34 = *E. coli* BL21(DE3) carrying pEG34;

BEG0 = *E. coli* BL21(DE3) carrying empty plasmid pEG0

sX.Z#Y = E. coli BL21(DE3) carrying pEG34, having wt folP promoter swapped for burden addiction-promoter X, wherein X refers to the promoter IDs in Table 2. The number Y following # indicates the selected clone from the pool of four RBS variants introduced by degenerate primer (Table 7). Z refers to the production genes for hGH-GFP (0 or 6), mevalonic acid (1), lysostaphin (4), respectively.

**Table 7:** Identity of the "N" nucleotide of the RBS of the respective *E. coli* strains

| Strain identifier | RBS "N" identity | Strain identifier | RBS "N" identity | Strain identifier | RBS "N" identity |
|---|---|---|---|---|---|
| s5.0 #3 | C | s16.6#6 | A | s19.6#2 | A |
| s7.0 #8 | A | s18.6#8 | T | s21.6#8 | C |
| s9.0#8 | NGS | s9.1#1 | C | s23.6#4 | T |
| s6.4#5 | C | s15.1#1 | A | s24.6#2 | T |
| s3.6#2 | A | s19.1#1 | T | s25.6#1 | C |
| s6.6 #6 | C | s14.1#5 | T | s29.6#3 | T |
| s7.6#8 | T | s15.1#5 | A | s22.6#8 | T |
| s10.6#7 | A | s19.1#7 | A | s26.6#3 | A |
| s13.6#2 | C | s15.6#1 | A | s29.6#4 | T |
| s15.6#7 | A | s16.6#7 | T | s30.6#6 | T |

*2.2 Results*

2.2.1 Short term hGH-GFP productivity screening

The productivity of four hGH-GFP producing strains comprising burden-sensing promoters selected from a group of heat shock promoters ($p_{ibpA}$, $p_{grpE}$, $p_{ycjX}$, and $p_{ybbN}$) and having one of four RBS coding sequence variants was tested under simulated large-scale production conditions, as follows. The strains were serially passaged by 100x back-dilution every day for 6 consecutive days corresponding to ca. 6 generation per seed. By day 6 (seed 6), several strains showed an elevated hGH-GFP synthesis compared to the non-burden addicted production strain BEG34, both during seed 1 and seed 6. The strains having high short term hGH-GFP productivity relative to strain BEG34 are s3.6#2 ($p_{ibpA}$), s6.6#6 ($p_{grpE}$), s7.6#8 ($p_{ycjX}$), and s10.6#7 ($p_{ybbN}$) - see figure 5.

2.2.2 Enhanced long term hGH-GFP productivity

hGH-GFP productivity of burden-addicted strains, s3.6#2, s6.6#6, s7.6#8, s10.6#7, and non-burden addicted strains BEG34 and non-producing strain BEG0 was monitored under simulated large-scale fermentation by serial transfer. Following seed 2, burden-addicted strains s3.6#2, s6.6#6, s7.6#8, s10.6#7 and the non-burden addicted control strain BEG34 performed equally well in terms of hGH-GFP synthesis (figure 6. However, after an additional ca. 20 generations (seed 4), hGH-GFP productivity by the control strain, BEG34, had declined considerably; and by seed 6, the strain essentially ceased production. Surprisingly, the burden-addicted strain s7.6#8 (with $p_{ycj}x$ controlling *folP-glmM*) still retained a productivity of around 50 % of seed 2 level. Additionally, burden-addicted strains s3.6#2 (with $p_{ibp}A$ controlling *folP-glmM*), s10.6#7 (with $p_{ybbN}$ controlling *folP-glmM*), and s6.6#6 (with $p_{grpE}$ controlling *folP-glmM*) also showed significantly better long-term productivity as compared to the non-burden addicted strain BEG34.

2.2.3 Growth rate of burden addicted strains

The growth rates of hGH-GFP producing burden-addicted strains s3.6#2, s6.6#6, s7.6#8 and s10.6#7 is no higher than the non-burden addicted hGH-GFP production strain BEG34; and hence their improved hGH-GFP production levels over time does not to simply stem from a reduced in initial production level and thus inherently lower burden, (figure 7).

2.2.4 Dependence of burden addiction genetic circuit on selected essential gene

A hGH-GFP producing burden-addicted strain (s13.6#2 (fxsA)) comprising the heat shock promoter $p_{fxsA}$ controlling the essential gene *folP-glmM* was compared with a mutant derivative comprising a frame-shift *folP* abolishing folP expression, strain s13.6#2evo (fxsA), whose growth was solely dependent on glmM expression. Although the loss of folP expression led to a lower growth rate (data not shown) in complex 2xYT medium, the essential gene *glmM,* alone is shown to be sufficient to confer enhanced long-term hGH-GFP production stability in the burden-addicted strain s13.6#2evo(fxsA) at levels comparable to the strain s7.6#8 (pycjX), when compared to the non-burden addicted hGH-GFP producing strain BEG34 (figure 8).

2.2.5 Use of ribosomal RNA promoters in the burden addiction genetic circuit

In addition to heat-shock promoters, ribosomal RNA promoters such as $p_{rrnB}$ and $p_{rrnE}$ are shown to be capable of sensing a burden-addicted state in a cell and, in response, to control essential gene expression such as to enhance long-term productivity in a hGH-GFP production strain. As seen in figure 9, the long-term stability of hGH-GFP production in strains s15.6.7 ($p_{rrnB}$) and s16.6.6 ($p_{rrnE}$), was significantly improved when compared to a non-burden addicted hGH-GFP producing *E. coli* strain BEG34, under simulated large scale production corresponding to around 110 generations of cell division.

2.2.6 Use of oxidative stress sensing promoters in the burden addiction genetic circuit

Oxidative stress-sensing promoters such as $p_{poxB}$ are shown to be capable of sensing a burden-addicted state in a cell and, in response, to control essential gene expression such as to enhance long-term productivity in a hGH-GFP production strain. As seen in figure 10, both production levels and long-term stability of hGH-GFP production in strain s29.6#3 ($p_{poxB}$), was significantly increased when compared to a non-burden addicted hGH-GFP producing *E. coli* strain BEG34, under simulated large scale production of seven serial passages corresponding to approximately 90 generations and time-extended cultivation for the final seed. As seen in figure 10, production using the burden addiction genetic circuit based on $p_{poxB}$ surprisingly resulted in elevated production over time, indicative of the enrichment of non-genetically high performing variants in the population.

*In summary,* this example illustrates that burden addiction enhances long-term stability and production in *E. coli* strains engineered to synthesize human growth hormone fused to GFP by coupling transcription of essential genes *folP-glmM* to any one of a *E. coli* heat shock, oxidative stress and DNA damage responsive promoter as well as an rRNA promoter.

**Example 3: Burden addicted *E. coli* strains producing lysostaphin show enhanced long-term production stability**

Lysostaphin is a 27 kDa endopeptidase that cleaves crosslinking pentaglycine bridges in the cell wall peptidoglycan of *Staphylococcus aureus* resulting in cell lysis. This antibacterial agent can be synthesized recombinantly in *E. coli.* The burden-addiction genetic circuit comprising a promoter having burden-sensing properties is shown to enhance the production stability of such *E. coli* strains engineered to synthesize lysostaphin, as illustrated by the following burden-addicted *E. coli* strains producing lysostaphin under simulated large-scale production conditions. The productivity of the burden-addiction production strains is further shown to be optimized by selecting a strain where the burden-sensing promoter is combined with an RBS sequence (Table 6) conferring an optimized translational strength of the cognate essential gene. These advantages are exemplified by burden-addicted strain s6.4#5 ($p_{grpE}$), described below, that is shown to be more resilient to loss of production due to addiction to the burden or fitness cost resulting from lysostaphin expression.

*3.1 Materials and Methods*

*Burden-sensing promoters:* A promoter integration sequence comprising the promoter $p_{grpE}$ was generated as described for example 1.2.1, by amplifying the $p_{grpE}$ promoter by PCR, using the $p_{grpE}$-specific primers specified in Table 6.

*Chromosomal integration of burden-addiction promoters:* the *grpE* promoter was integrated upstream of the *folP-glmM* gene in the genome of *E. coli* strain BENDU5cam (corresponding to *E. coli* BL21(DE3) harboring a lysostaphin producing plasmid pENDU5cam), as follows. Cells of the *E. coli* strain BENDU5cam, pre-transformed with the recombineering plasmid pSIM5-tet, was prepared and transformed by electroporation with the $p_{grpE}$ promoter integration sequence, as described in example 1.2.1. Following the described steps of recombineering and curing of pSIM5-tet, 5 colonies were picked, corresponding to clones with the same promoter integration but having a random variant RBS sequence (see Table 6). Each clone was then transferred to a 15 mL Greiner culture tube containing 4 mL 2xYT supplemented with chloramphenicol for maintenance of the plasmid, pENDU5cam, and cultured. 2 µl of each cultured clone was used to validate promotor integration as described in example 1.2.1; prior to freezing.

*Lysostaphin production screening*: The *E. coli* strain s6.4#5($p_{grpE}$) having the highest lysostaphin-productivity was identified using a screening assay in which the three other potential combinations of RBS and $p_{grpE}$ were cultured in 2xYT supplemented with chloramphenicol in 96-well format over six serial passages of 1000x dilution (corresponding to 60 generations) as compared to the non-burden addicted production strain BENDU5cam. The strains were grown in 3mL 2xYT media containing 30 mg/L chloramphenicol in 15 mL culture tubes at 37°C for 21 hours. 150 µL of the cultures were mixed with 50 µl 50% glycerol and stored in a 96-well plate at -80°C. Additionally, 30 µL of the cultures were transferred to 3 ml fresh 2xYT media supplemented with chloramphenicol and grown for another 21 hours at identical conditions; and repeated for a total of 5 transfers where freeze-stocks were made of the overnight cultures with each transfer.

*Lysostaphin expression and detection assay*: The 96-well plate containing all freeze-stocks from the 5 transfers were thawed on ice. 20 µL from each well were transferred to a 96-well plate containing 180 µL 2xYT supplemented with chloramphenicol, and cultured grown at 37°C in a plate reader until most wells had reached OD630=0.35; and then 10 µl 2xYT supplemented 30 mg/L chloramphenicol and 20 mM IPTG was added to each well to a final concentration of 1 mM IPTG, sufficient to induce lysostaphin gene expression. The induced cultures were grown at 37°C for 4 hours, and the plate was then centrifuged at 4000 RPM for 10 minutes. 100 µl supernatant was transferred to a new 96-well plate containing 100 µl overnight *S. aureus* culture. The *S. aureus* lysis in each well was monitored at OD630 on a plate reader at 37°C for 2 hours every 10 minutes. The *S. aureus* lysis rate was quantified by dividing the change in *S. aureus* OD by the timeframe of 60 minutes that the change occurred in. The equation can be seen below:

$$Rate_{lysis} = \frac{OD_{t_0} - OD_{t_1}}{t_1 - t_0}$$

The specific lysostaphin synthesis rate was determined by normalizing the measured rate to the final OD630 measurement of the respective production *E. coli* culture.

*3.2 Results*

The burden-addicted lysostaphin-producing strains having a $p_{grpE}$ promoter and one of four RBS coding sequence variants controlling expression of the essential genes, *folP-glmM,* were cultivated under simulated large-scale production conditions, achieved by serial passaging. As seen in figure 11, strain s6.4#5 ($p_{grpE}$) maintained significantly higher levels of lysostaphin production as compared to the steep reduction in production seen in the non-burden addicted lysostaphin-production BENDU5cam strain. Since the initial lysostaphin production rates for s6.4#5 ($p_{grpE}$) and BENDU5cam strains at seed 0 were the same; the smaller decline in production rates for s6.4#5 ($p_{grpE}$) is not due to an inherently lower initial burden from lysostaphin production.

*In summary*: Burden-addiction was demonstrated to enhance long-term stability and production in *E. coli* engineered to synthesize secreted lysostaphin by coupling essential genes *folP-glmM* transcription to the *E. coli* heat shock promoter $p_{grpE}$.

**Example 4: Burden addicted *E. coli* strains producing mevalonic acid show enhanced long-term production stability**

E. coli BL21(DE3) cells were engineered to synthesize mevalonic acid by introducing a plasmid pMevT expressing a heterologous three-step enzymatic pathway (Martin et al., 2003) that converts glucose to mevalonic acid via the acetyl-CoA pool. A burden-addiction genetic circuit comprising a promoter having burden-sensing properties and controlling transcription of an essential gene is shown to enhance the production stability of such mevalonic acid-producing *E. coli* strains under simulated large-scale production conditions. The productivity of the burden-addiction production strains is shown to be further optimized by selecting a strain where the burden-sensing promoter in combined with a RBS coding sequence (Table 5) conferring an optimized translational strength of the cognate essential gene.

Specifically, mevalonic acid-production strains comprising the burden-addiction promoter, $p_{cspD}$ (an oxidative stress and glucose starvation sensing promoter), or $p_{mutM}$ (heat shock/DNA damage-sensing promoter) controlling *folP-glmM* transcription, are both shown to be more resilient to loss of production than the non-burden addicted strain due to their addiction to transcriptional signals resulting from mevalonic acid production.

*4.1 Materials and Methods*

*Burden-sensing promoters:* Promoter integration sequences comprising the promoters $p_{mutM}$ and $p_{cspD}$ were generated as described for example 1.2.1, by amplifying the $p_{mutM}$ and $p_{cspD}$ promoters by PCR, using the $p_{mutM}$- and $p_{cspD}$-specific primers specified in Table 8.

| Table 8 Primers | | | |
|---|---|---|---|
| Promoter of *E. coli* gene | Primer name | Primer sequence (5'-3')* | SEQ ID No.: |
| *mutM* | mutM for | ATTCATATGUGGCGAAAAGTACTAAGTACTTA | 116 |
| | mutM rev | ATCCCCATTACGTGAGGATGGCTAAGGTCCAGTGAA GTACCCTGGGCAAAGAGTTTCATaccttNCAAGTAGC ATCTCCAGGAATGAACA | 117 |
| *cspD* | cspD fwd | ATTCATATGUCGGTTATCGGCAGAACGCCCTG | 138 |
| | cspD rev | ATCCCCATTACGTGAGGATGGCTAAGGTCCAGTGAA GTACCCTGGGCAAAGAGTTTCATaccttNCAAGTGCT TCGACATCCTTCGCAAA | 139 |
| * RBS complementary coding sequences are in bold font; wherein N is either A, G, T or C. The RBS is encoded on the opposite strand of the oligo sequence shown. | | | |

*Chromosomal integration of burden-addiction promoters:* the $p_{mutM}$ and $p_{cspD}$ were individually integrated upstream of the *folP-glmM* gene in the genome of cells of the *E. coli* strain BL21(DE3)pMevT, harboring a lysostaphin producing plasmid pMevT, as follows. Cells of the *E. coli* strain BL21(DE3)pMevT, pre-transformed with the recombineering plasmid pSIM5-tet, were prepared and transformed by electroporation with each of the promoter integration sequences, as described in example 1.2.1. Following the described steps of recombineering and curing of pSIM5-tet, 5 colonies were picked, corresponding to clones with the same promoter integration but having a random variant RBS sequence (see Table 8). Each clone was then transferred to a well of a 96-well plate containing 200 $\mu$l 2xYT supplemented with chloramphenicol for maintenance of the plasmid, pMevT, and cultured. 2 $\mu$l of each cultured clone was used to validate promotor integration as described in example 1.2.1; prior to freezing the 96-well plate.

*Mevalonic acid production screening:* The *E. coli* strains s19.1.1($p_{cspD}$) and s9.1.4($p_{mutM}$) having the highest mevalonic acid productivity were identified using a screening assay in which strains comprising the four other potential combinations of RBS and promoter were cultured in 96-well plates over six serial passages of 1000x dilution (corresponding to 60 generations) in parallel with the non-burden addicted production *E. coli* strain BL21(DE3)pMevT, as follows. The strains were grown in 200 $\mu$L 2xYT media containing 30 mg/L chloramphenicol and 0.5mM IPTG in microtiter plate sealed with breathe-easy seal at 37°C for 21 hours with horizontal shaking. 150 $\mu$L of the cultures were mixed with 50 $\mu$l 50% glycerol and stored in a 96-well plate at -80°C. Additionally, 2 $\mu$l of a 10-fold diluted culture was transferred to 200 $\mu$L fresh 2xYT media supplemented with chloramphenicol and 0.5mM IPTG and grown for another 21 hours under identical conditions. In total, 5 transfers identical to the one described above were made and freeze-stocks were made of the overnight cultures with each transfer.

*Mevalonic acid synthesis and detection assay:* The 96-well plate containing freeze-stocks from the second, fifth and sixth transfers were thawed on ice and used to inoculate 10 mL 2xYT with 0.5 mM IPTG and 30 mg/L chloramphenicol and cultivated at 37°C with horizontal shaking (250 rpm) for 54 hours. 300 $\mu$L aliquots from each culture were treated with 23 $\mu$L 20% sulfuric acid; vigorously shaken and then spun down at 13 000 $\times$ g for 2 min. Supernatant (medium) samples were injected into an Ultimate 3000 high-performance liquid chromatography running a 5 mM sulfuric acid mobile phase (0.6 mL/min) on an Aminex HPX-87H ion exclusion column (300 mm $\times$ 7.8 mm, Bio-Rad Laboratories) at 50 °C. A refractive index detector was used for detection. A standard curve for mevalonic acid was generated with mevalonolactone (Sigma-Aldrich) dissolved in 2xYT medium supernatant of a non-producing *E. coli* strain incubated under the same conditions.

*4.2 Results*

The burden-addicted mevalonic acid-producing strains having the $p_{cspD}$ or $p_{mutM}$ promoters and one of four RBS coding sequence variants controlling expression of the essential genes, *folP-glmM,* were cultivated under simulated large-scale production conditions, achieved by serial passaging. As seen in figure 12, both selected burden-addicted strains s19.1.1($p_{cspD}$) and s9.1.4($p_{mutM}$) retained significantly higher levels of mevalonic acid production as compared to the steep reduction in production seen in the non-burden addicted mevalonic acid-production strain BL21(DE3)pMevT. Since the initial mevalonic acid production rates for each burden-addicted strain was the same and the control BL21(DE3)pMevT strain at seed 0; the smaller decline in production rates for strains s19.1.1($p_{cspD}$) and s9.1.4($p_{mutM}$) is not due to an inherently lower initial burden from mevalonic acid production.

*In summary*: Burden-addiction is demonstrated to enhance long-term stability and production in *E. coli* engineered to synthesize mevalonic acid by coupling essential genes *folP-glmM* transcription to the *E. coli* oxidative stress and glucose starvation sensing promoter $p_{cspD}$, and the heat shock/DNA damage-sensing promoters $p_{mutM}$.

**Example 5: Evaluation of promoters as burden sensors to render yeast cells addicted to the burden of engineered recombinant protein production**

Promoters capable of sensing the burden or load on a cell brought about by recombinant expression of a protein or a biosynthetic pathway, and that then induce the expression of an essential gene, can be used to create a burden addiction genetic circuit tailored for use in yeast. A method for evaluating candidate promoters is illustrated in yeast cells genetically engineered to synthesize recombinant human serum albumin (hSA) or insulin precursor (IP), optionally translationally fused to green fluorescent protein (GFP).

To render growth responsive to the activity of a candidate burden-sensing promoter, the native promoter of an essential gene in the yeast *Saccharomyces cerevisiae* is genetically replaced with a candidate promoter using homologous recombination of linear DNA constructs transformed into the yeast cells using a standard selectable marker. By way of example, candidate promoters may be selected from upregulated promoters of ribosomal RNA genes, such as those transcribed by RNA polymerase I (Laferté et al., 2006), DNA damage sensing promoters (e.g. pOGG1), and unfolded protein response (UPR) promoters upregulated by the *HAC1* transcription factor (Kimata et al., 2006). In order to ensure that the burden-sensing promoter, once activated, confers the cell with a selective growth advantage compared to a non-sensing promoter, it may be necessary to fine-tune the expression level of essential gene. A range of translational strengths can be engineered by varying the translation initiation region introduced with the burden-sensing promoter.

Clones with potentially different combinations of candidate burden-sensing promoters are selected and evaluated for maintained protein production over 30-100 generations of cell division.

*5.1 Materials and methods*

*Growth medium*: YPD medium comprises 1% yeast extract, 2% peptone, 2% glucose. SC medium comprises 6.7 g/L yeast nitrogen base without amino acids and with ammonium sulfate, but lacks uracil.

*Chromosomal integration and validation of promoter constructs*: Constructs for chromosomal integration will contain 300-600 bp upstream of the natively regulated gene. Chromosomal integration of the promoter construct is performed by transformation and homologous recombination using standard electroporation procedures for *S. cerevisiae.* Correct chromosomal integration of the promoter was validated using colony PCR.

*Long-term cultivation and production*: A single colony from each strain is transferred to 24-deep well plates and cultivated in 1.8 mL YPD medium under conditions inducing recombinant protein production, at 250 rpm and 30 °C. Following 48 hours of cultivation, cells are passaged to a new deep-well plate under identical conditions by 1000x back-dilution. Samples are analyzed for production using recombinant protein specific assays (e.g. GFP detection) and the cell density is monitored by OD600.

*Growth rate measurements of selected strains*: Growth rates of individual strains are compared to non-burden addicted production strains. The 96-well plate is sealed with breathe-easy film and growth is measured in a Synergy H1 plate reader for 20 hours at 37°C and 754 rpm linear shaking. OD600 is measured every 10 minutes.

*5.2 Results*

5.2.1 HAC1-upregulated promoters

The HAC1-upregulated promoters comprising an unfolded protein response (UPR) element, e.g. *KAR2 (SEQ ID NO.: 91), PDI1 (SEQ ID NO.: 92)*, *SSA1 (SEQ ID NO.: 93)* or *FPR2 (SEQ ID NO.: 94),* are demonstrated to be useful in regulating growth when introduced in front of a native growth regulating gene (e.g. the conditionally essential gene *URA3* encoding orotidine 5'-phosphate decarboxylase essential for pyrimidine biosynthesis) in a recombinant protein production *Saccharomyces* strain producing human insulin precursor or human serum albumin, optionally coupled to GFP. Stability of production is followed under simulated long-term production via serial passages corresponding to 60-80 generations of cell division. In *Saccharomyces* strains comprising the *HAC1*-upregulated promoter controlling transcription of the growth-regulating gene (URA3), production is expected to be more stable than the corresponding parent recombinant protein production *Saccharomyces* strain.

5.3.2 RNA polymerase I upregulated promoters

RNA polymerase I transcribe ribosomal RNA genes in yeast. RNA polymerase I upregulated promoters such as promoters of the genes: *RPL3* (SEQ ID NO.: 95), *RPL6A* (SEQ ID NO.: 96) and *RPL28* (SEQ ID NO.: 97) are useful in regulating growth of yeast essential genes. Such upregulated promoters are introduced in front of native growth regulating gene (e.g. the conditionally essential gene *URA3)* in a recombinant protein overproduction strain producing human insulin precursor or human serum albumin, potentially coupled to GFP. Stability of production is followed by experimentally simulated long-term production via serial passages corresponding to 60-80 generations of cell division. In *Saccharomyces* strains comprising an RNA polymerase I upregulated promoter controlling transcription of the conditionally essential gene, *URA3,* production will be more stable than the corresponding parent recombinant protein production *Saccharomyces* strain.

5.3.3 DNA damage responsive promoters

DNA damage response in yeast, resulting from heterologous expression broadly induces transcription of the DNA repair system, including OGG1 *(SEQ ID NO.: 98), RAD51 (SEQ ID NO.:* 99) and *RAD54 (SEQ* ID *NO.: 100).* Promoters of the genes encoding OGG1, *RAD51* or *RAD54,* when operatively linked to an essential gene, are useful for regulating growth of a yeast production cell of the invention. Such promoters are introduced in front of native essential gene (e.g. the growth regulating gene encoding URA3) in cells of a yeast protein production strain producing human insulin precursor or human serum albumin, optionally fused to GFP.

Stability of production is followed by under simulated long-term production via serial passages corresponding to 60-80 generations of cell division. In strains with a $p_{RAD51}$ and/or $p_{RAD54}$-upregulated essential gene, production will be more stable.

*In summary*: Burden addiction enhances long-term stability and production in cells of budding yeast engineered to synthesize human serum albumin production or insulin precursor by coupling essential gene transcription to the burden-sensing promoters selected from promoters activated during burden of recombinant protein production or associated with ribosomal RNA promoters (Table 2).

**Example 6 Examples of burden-addicted yeast strains producing human serum albumin showing enhanced long-term production stability**

Burden-addiction systems for use in yeast strains include promoters derived from genes encoding: 1) Protein isomerase PDI1 encoding a chaperone belonging to the unfolded protein response of yeasts such as *S. cerevisiae* and *P. pastoris,* and whose abundance is frequently upregulated in response to overexpression of a recombinant protein; 2) Ribosomal subunits encoded by the *RPL6A* and *RPL3* genes and 3) *FPR2* encoding a peptidyl-prolyl cis-trans isomerase which is known to be activated upon DNA replication stress. Burden-addiction systems based on these promoters were introduced into a strain of *Pichia pastoris* engineered to express and secrete human serum albumin in order to determine their effect on long term human serum albumin (hSA) production stability.

*6.1 Materials and methods:*

The *Pichia pastoris* (*Komagataella phaffii*) strain EGS31 is a derivative of the CBS7435 strain (NRRL-Y11430 or ATCC 76273), engineered to secrete hSA by a genomically integrated cDNA version of the hSA coding *ALB1* gene under control of the *AOX1* promoter.

*Strain construction*: Burden-addicted versions of the EGS31 strain were generated by genetically integrating a construct comprising a kanMX conditionally selectable G418 resistance gene operably linked to one of the burden-responsive promoters: *pPDI1, pFPR2, pRPL3* and *pRPL6A*. These burden-addiction constructs were integrated into the KU70 genomic locus of the *P. pastoris* strain by transforming EGS31 cells with linear integration DNA (sequences N1-N3 respectively) flanked by >750 bp homology arms. Transformation was performed using a standard electroporation procedure on exponentially grown cells pretreated with lithium acetate and dithiothreitol (Wu & Letchworth, 2004).

| Table 9: Recombinant genes and burden-addiction constructs introduced into *P. pastoris* | | |
|---|---|---|
| Gene/construct name | Construct nucleotide SEQ ID NO: | Encoded protein SEQ ID NO.: |
| pAOX1-ALB construct | SEQ ID NO: 155 | hSA protein SEQ ID NO: 156 |
| KU70(up)-pPDI1-kanMX-terminator-KU70 (dw) | SEQ ID NO: 157 | kanMX protein SEQ ID NO: 158 |
| KU70(up)-pFPR2-kanMX-terminator-KU70 (dw) | SEQ ID NO: 159 | kanMX protein SEQ ID NO: 160 |
| KU70(up)-pRPL3-kanMX-terminator-KU70 (dw) | SEQ ID NO: 161 | kanMX protein SEQ ID NO: 162 |
| KU70(up)-pRPL6A-kanMX-terminator-KU70 (dw) | SEQ ID NO: 163 | kanMX protein SEQ ID NO: 164 |

*Growth Media*: BMGY and BMMY liquid media (1L) was prepared as follows: 10 g yeast extract and 20 g peptone was added to 700mL $H_2O$; mixed with a magnetic stirrer and then autoclaved. After cooling to room temperature; the following was added to the solution: 100ml 1M potassium phosphate buffer (pH 6.0); 100 ml 13.4% (w/v) Yeast Nitrogen Base with Ammonium Sulfate without amino acids; 2 ml 0.02% (w/v) biotin, and in the case of BMGY, 100 ml 10% (v/v) glycerol was added; and in the case of BMMY, 100mL 5% (v/v) methanol was added.

*Cultivation: EGS31* and burden-addicted EGS31 strains were streaked on YPD (1 % yeast extract, 2 % peptone, 2 % D-glucose) agar plates and incubated overnight at 30 deg. C. Single colonies were picked and pre-cultured in 2 mL BMGY where cultures of burden-addicted strains were supplemented 50 ug/mL G418 and cultured at 30 deg. C with 300 rpm horizontal shaking overnight. Expression cultures were seeded using 1 uL preculture into 500 $\mu$L BMMY medium containing different concentrations (0 $\mu$g/mL, 750 $\mu$g/mL) of G418 in a 96-well deep well plate with aerating lid to generate "seed 1".

The cultures were incubated at 30 deg. C for 72 hours (300 rpm horizontal shaking). To generate the next seed, four additional times, grown cultures were serially passaged (500x dilution) to new 500 $\mu$L BMMY medium containing different concentrations (0 $\mu$g/mL, 750 $\mu$g/mL) of G418 in a 96-well deep well plate with aerating lids. At each serial passage, glycerol stocks (20 % glycerol) were stored at -80 deg. C from the grown cultures.

To quantify production of secreted hSA, quantification cultures were re-grown from glycerol stocks in 500 $\mu$L BMMY medium containing different concentrations (0 $\mu$g/mL and 750 $\mu$g/mL) of G418 in a 96-well deep well plate with aerating lid for 72 hours.

Cultures were centrifuged at 3000 g for 15 minutes and the concentration in 50 $\mu$L supernatants was quantified using a hSA-specific ELISA kit (Abcam catalog no: ab179887: Human Albumin SimpleStep ELISA® Kit) following manufacturer's instructions.

*6.2 Results*

Strains with a genomically integrated burden addiction promoter, *PDI1,* operably linked to the selectable *kanMX* gene displayed higher production of secreted hSA when strain's burden-addiction system was activated by addition of G418 (figure 13). Further, improved hSA production was seen with higher selection (750 $\mu$g/mL G418), for each of the other tested burden-addiction promoters after approximately 30 cell divisions (Figure 14A - Seed 1). When further cultivated for about an additional 10 cell divisions (Figure 14B -Seed 2) the strains show enhanced levels of hAS production when burden addiction is activated (150-750 $\mu$g/mL G418) compared to the non burden-addicted control and strains without burden addiction activated (0 $\mu$g/mL G418).

*In conclusion,* the cultivation of yeast cells comprising the exemplified burden-addiction systems of the invention, under conditions that activate their respective burden-addiction system, is believed to enrich for high-producing yeast variants within the cultured population. While an increase in hSA production may be detectable after relatively short cultivation (30 cell divisions), enrichment for high-producing yeast variants is both maintained and further enhanced over longer cultivation periods when traditional cultures commonly exhibit a significant decline in productivity.

**Example 7 Identification of suitable burden sensing promoter candidates**

Different engineered production genes and pathways elicit different transcriptional responses indicative of the production process. In order to identify suitable promoter candidates for use as burden sensors, the following experiment was conducted.

*Methods:* Typical genetic escaper cells were isolated from long-term cultivation with the genetically engineered microbial production cells of interest in the intended fermentation medium. Suitable genetic escaper cells are characterized by having at least 5 % higher exponential-phase growth rate and at least 30 % lower production rate or product yield than the original genetically engineered production cell.

Production cells and corresponding escaper cells were cultured under intended fermentation conditions, scaled down conditions or shake flask conditions mimicking intended fermentation conditions. At time points corresponding to the highest rate of production in the production cells, samples were taken for RNA sequencing. Total RNA was purified using Purelink RNA Mini kit (Thermo Fischer) and prepared using TruSeq Stranded mRNA kit (Illumina) following the kit manufacturer's instructions. Reads were mapped and analyzed to the reference genome of the strain and next analyzed for differential expression between the production cells and corresponding escaper cells. *Results*: Candidate suitable promoters were identified as those driving expression of genes that showed a differential expression of >3 fold higher expression in the production organism relative to at least one isolated genetic escape strain.

### Example 8: Burden addicted *Bacillus subtilis* strains producing IgA fragment show enhanced long-term production stability

Burden-sensing promoters are shown to be promoters that can sense and be activated by a burden-induced state in a cell resulting from the cell's synthesis of a recombinant protein, an IgA fragment C-terminally fused to GFP (IgA-GFP). Once activated, burden-sensing promoters are shown to elevate expression of essential gene (operons), *iscU* or *accC,* to a level sufficient to confer a selective growth advantage on a cell when compared to a non-productive cell. In order to maximize the dynamic range of essential gene expression in response to its cognate burden sensing promoter, the burden-sensing promoter is randomly combined with variant RBS coding sequences (Table 3.2) conferring different translational strengths.

Promoters having burden-sensing properties suitable for use in a burden-addiction genetic circuit are shown to include heat-shock-, DNA damage-, and oxidative stress response-promoters, as illustrated by the following engineered production strains cultured under simulated large-scale production conditions.

### 8.1 Materials and Methods

Burden-sensing promoters: The promoters, $P_{hrcA}$ $P_{perR}$ and their respective integration sequences to target essential genes were generated by PCR and USER cloning resulting in integration vectors (Table 10).

Table 10: Bacillus chromosomal integration constructs for inserting burden-sensing promoter to control essential genes.

| Plasmid | Promoter-essential gene | Resistance gene used to select for chromosomal integration |
|---|---|---|
| pEG151 (SEQ ID NO. 195) | $P_{groES}$-*iscU* | specR (SEQ ID No. 211) |
| pEG152 (SEQ ID NO. 196) | $P_{ctsR}$-*iscU* | specR (SEQ ID No. 211) |
| pEG153 (SEQ ID NO. 197) | $P_{dnaK}$-*iscU* | specR (SEQ ID No. 211) |
| pEG154 (SEQ ID NO. 198) | $P_{perR}$-*iscU* | specR (SEQ ID No. 211) |
| pEG155 (SEQ ID NO. 199) | $P_{hrcA}$-*iscU* | specR (SEQ ID No. 211) |
| pEG156 (SEQ ID NO. 200) | $P_{spx}$-*iscU* | specR (SEQ ID No. 211) |
| pEG157 (SEQ ID NO. 201) | $P_{sigB}$-*iscU* | specR (SEQ ID No. 211) |
| pEG158 (SEQ ID NO. 202) | $P_{yflT}$-*iscU* | specR (SEQ ID No. 211) |
| pEG159 (SEQ ID NO. 203) | $P_{groES}$-*accC* | specR (SEQ ID No. 211) |
| pEG160 (SEQ ID NO. 204) | $P_{ctsR}$-*accC* | specR (SEQ ID No. 211) |
| pEG161 (SEQ ID NO. 205) | $P_{dnaK}$-*accC* | specR (SEQ ID No. 211) |
| pEG162 (SEQ ID NO. 206) | $P_{perR}$-*accC* | specR (SEQ ID No. 211) |

(continued)

| Table 10: Bacillus chromosomal integration constructs for inserting burden-sensing promoter to control essential genes. | | |
|---|---|---|
| **Plasmid** | **Promoter-essential gene** | **Resistance gene used to select for chromosomal integration** |
| pEG163 (SEQ ID NO. 207) | P$_{hrcA}$-accC | specR (SEQ ID No. 211) |
| pEG164 (SEQ ID NO. 208) | P$_{spx}$-accC | specR (SEQ ID No. 211) |
| pEG165 (SEQ ID NO. 209) | P$_{sigB}$-accC | specR (SEQ ID No. 211) |
| pEG166 (SEQ ID NO. 210) | P$_{yflT}$-accC | specR (SEQ ID No. 211) |
| pEG204 | P$_{groEs}$-accC | camR (SEQ ID No. 220) |
| pEG205 | P$_{ctsR}$-accC | camR (SEQ ID No. 220) |
| pEG206 | P$_{dnaK}$-accC | camR (SEQ ID No. 220) |
| pEG207 | P$_{perR}$-BCCC | camR (SEQ ID No. 220) |
| pEG208 | P$_{hrcA}$-accC | camR (SEQ ID No. 220) |
| pEG209 | P$_{spx}$-accC | camR (SEQ ID No. 220) |
| pEG210 | P$_{sigB}$-accC | camR (SEQ ID No. 220) |
| pEG211 | P$_{yflT}$-accC | camR (SEQ ID No. 220) |

| Table 11 List of *Bacillus* strains investigated | | | | |
|---|---|---|---|---|
| **Strain name** | **New features** | **Transformed with** | **Last nucleotide in RBS of new targeted essential gene** | **Parental strain** |
| *B. subtilis* KO7 | - | - | - | - |
| EGS084 | IgA-GFP expression | pEG062 (SEQ ID NO. 222) | - | *B. subtilis* KO7 |
| EGS340 | P$_{perR}$-*iscU* | pEG154 | T | EGS084 |
| EGS343 | P$_{perR}$-*accC* | pEG162 | T | EGS084 |
| EGS460 | P$_{ctsR}$-*accC* | pEG205 | C | EGS340 |
| EGS462 | P$_{dnaK}$-*accC* | pEG206 | G | EGS340 |
| EGS466 | P$_{hrcA}$-accC | pEG208 | T | EGS340 |

Chromosomal integration of burden-addiction promoters: To construct the candidate burden-addicted strains (Table 11), integration vectors (Table 10) featuring each of the promoters fused to RBS variants (Table 3.2) were integrated by homologous recombination upstream of the operons containing either *iscU* or *accC* in the genome of the IgA-GFP producing EGS084 strain (corresponding to *B. subtilis* KO7 containing the expression cassette of pEG062 inserted in the *amyE* locus) as follows. Competent cells were prepared according to standard *Bacillus* transformation methods following a previously described protocol (dx.doi.org/10.17504/protocols.io.bdmti46n) and transformed with pEG151 to pEG166. The transformants were selected on LB agar plates supplemented with spectinomycin (200 μg/mL) and confirmed by colony PCR using the primers E257/E258 (*iscU*) or E261/E262 (*accC*).

| Table 12 Primers | | |
|---|---|---|
| SEQ ID NO. | Oligo ID | Sequence (5'-) |
| 223 | E257 | CTGAGTCAATTTCATCAAGG |
| 224 | E258 | CAGAAGAAAACATTCTCGAAGC |
| 225 | E261 | GAGGTCGCAACTGAAAAAGC |
| 226 | E262 | ATGTTCAGTCTCCTTACTCC |

Long term IgA-GFP production assay: A single selected colony from each burden-addicted IgA-GFP producing strain was used to inoculate a 96 deep-well plate containing 500 μL/well Cal18-2 media (dx.doi.org/10.17504/protocols.io.bdmui46w) supplemented with kanamycin (10 μg/mL). The cultures were grown for 24 hours at 30°C/250 RPM horizontally shaking incubator (Innova, 2-inch amplitude). 1 μL of each culture were then transferred (as serial passage) in 499 μL fresh Cal18-2 media (corresponding to approx. 10 generations per transfer) into a new deep well plate under identical cultivation conditions. These method steps were repeated for a total of up to 13 times. At each serial passage, 100 μL of each strain were banked in equal amount of 50% glycerol for storage and follow-up examinations. To quantify IgA-GFP expression after each passage, cultures were grown for an additional 24 h at the previously described growth conditions. The cultures were spun down at 2000 g for 5 min and washed in 1 volume PBS twice. 20 uL samples of the culture were added to 180 μl PBS (10x dilution) in a 96-well plate and the cell density and IgA-GFP production was quantified by measuring OD600 and GFP fluorescence ($\lambda_{ex}$/ $\lambda_{ex}$ = 485nm/528nm) in a Synergy H4 plate reader (Biotek). The specific production level of each culture was quantified as the GFP signal normalized to the OD600 value followed by subtraction of the same value measured in a non GFP-producing Bacillus subtilis KO7 strain.

*8.1 Results*

Following long-term cultivation of five 500-fold dilution serial passages (approx. 65 cell generations), IgA-GFP production was quantified and shown to be improved in the burden addicted strains (Figure 15 and 16).

Based on the methodology, similar burden-addicted strains can be constructed using other candidate burden sensors that may match the transcriptional burden response of other production strains. Their suitability can easily be assessed using the devised long-term production assays or for example in continuous small-scale (e.g. 400 mL) production cultures.

**Example 9: Burden addiction controlling multiple essential genes improves production**

It is here demonstrated how to introduce two (and by analogy further) burden-sensing promoters controlling different essential gene (operon)s in a single production cell to increase production of desired products and further prolong the period during which the production cells are productive Additionally, by using more than one burden-sensor to control essential genes, the transcriptional space that directs the imposed addiction-based selection regime can be further controlled.

The burden-addicted IgA-GFP production strain EGS340 (P$_{hrcA}$ controlling the *iscU* essential gene operon) was genetically transformed to exchange the native promoter of the *accC* essential gene operon for the candidate burden sensors P$_{ctsR}$, P$_{dnaK}$ and P$_{hrcA}$ respectively.

*9.1 Materials and methods*

Chromosomal integration of burden-addiction promoters: The spectinomycin resistance marker was exchanged for a chloramphenicol resistance marker in pEG159-pEG166 using the primers E372/E373 (for the vector backbones) and E374/E375 (for the chloramphenicol resistance marker in pDG1662, (Guerout-Fleury et al 1996) to create pEG204 to pEG211 (Table 10). Each of the promoters were integrated upstream of the essential gene operons containing *accC* in the genome of the EGS340 strain (corresponding to EGS084 containing P$_{perR}$ regulating the *iscU* essential gene operon) as follows. Competent cells were prepared according to a previously described method (dx.doi.org/10.17504/protocols.io.bdmti46n) and transformed with pEG204 to pEG211. The transformants were selected on LB agar plates supplemented with chloramphenicol (5 μg/mL) and confirmed by colony PCR using the primers E261/E262.

| Table 13 Primers | | |
|---|---|---|
| SEQ ID NO | Oligo ID | Sequence (5'-) |
| 227 | E372 | ATTATAACAUGTATTCACGAACGAAAATCG |
| 228 | E373 | ATCAGATTTUAGAAAACAATAAACCCTTGCATAGG |
| 229 | E374 | AAAATCTGAUACGGGGCAGG |
| 230 | E375 | ATGTTATAAUACCCATTAGTTCAACAAACGAAAATTG |

Long term IgA-GFP production assay: A single selected colony from each burden-addicted IgA-GFP producing strain was used to inoculate a 96 deep-well plate containing 500 μL/well Cal18-2 media (dx.doi.org/10.17504/protocols.io.bdmui46w) supplemented with kanamycin (10 μg/mL). The cultures were grown for 24 hours at 30°C/250 RPM horizontally shaking incubator (Innova, 2-inch amplitude). 1 μL of each culture were then transferred (as serial passage) in 499 μL fresh Cal18-2 media (corresponding to approx. 10 generations per transfer) into a new deep well plate under identical cultivation conditions. These method steps were repeated for a total of up to 13 times. At each serial passage, 100 μL of each strain were banked in equal amount of 50% glycerol for storage and follow-up examinations. To quantify IgA-GFP expression after each passage, cultures were grown for an additional 24 h at the previously described growth conditions. The cultures were spun down at 2000 g for 5 min and washed in 1 volume PBS twice. 20 uL samples of the culture were added to 180 μl PBS (10x dilution) in a 96-well plate and the cell density and IgA-GFP production was quantified by measuring OD600 and GFP fluorescence ($\lambda_{ex}$/ $\lambda_{ex}$ = 485nm/528nm) in a Synergy H4 plate reader (Biotek). The specific production level of each culture was quantified as the GFP signal normalized to the OD600 value followed by subtraction of the same value measured in a non GFP-producing Bacillus subtilis KO7 strain.

Cell disruption and ELISA: Following washing in PBS, the cultures were spun down at 2000g for 5 min. The pellets were resuspended in 1 volume of lysis buffer (10 mM Tris, pH 7.5, 150 mM NaCl, 500 μM EDTA), spun down again at 6000 g for 5 min, and resuspended in 1 volume of lysis buffer supplemented with lysozyme (10 mg/mL). The cells were disrupted by incubating the reactions for 30 min at 37 °C. Afterwards, the reactions were briefly vortexed and spun down at 12000 g for 30 min at 4 °C. The supernatants were subjected to ELISA using the Pig IgA ELISA Kit from Abcam (ab190536) according to manufacturer's protocol, except that after adding the chromogenic substrate, the absorbance of each well was read at 600 nm every 40 s for 10 min. The relative IgA concentrations were calculated as the slopes of values per OD600 of the original cultures.

*9.2 Results*

The single burden-sensor strain EGS340 carries only the $p_{perR}$-based burden sensor controlling transcription of the *iscU* essential gene operon (EGS340). EGS340 is compared to derived strains in which transcription of the *accC* essential gene operon is also regulated by respectively a $p_{ctsR}$-based burden sensor (EGS460), a $p_{dnaK}$-based burden sensor (EGS462), and a $p_{hrcA}$-based burden sensor (EGS466). Following long-term cultivation for approx. 75 cell generations, IgA-GFP production was quantified and shown to be improved in the burden addicted strains (Figure 17 and 18).

Based on the methodology, similar burden-addicted strains can easily be constructed using other candidate burden sensors that may match the transcriptional burden response of other production strains. Their suitability can easily be assessed using the devised long-term production assays or in continuous small-scale (e.g. 400 mL) production cultures.

**Example 10: Additional method for identification of suitable burden-sensing promoters**

In this example, suitable candidate burden-sensing promoters are identified for use in burden addiction to sustain long-term heterologous production in particular production strains.

The method is divided in an optional track 1 for *de novo* discovery (RNA-sequencing) and a track 2 for confirmation of putative burden-sensing promoters (q-PCR).

In track 1, using RNA-sequencing the transcriptional activity of coding genes in the production organism during typical production conditions (in terms of e.g. temperature, stirring, product/by-product/substrate concentration/growth phase) is compared to that of a an isolated non/low-producing organism during the same typical production conditions. The culture of the isolated non/low-producing organism may optionally be fed with product to the concentration approximately found in a corresponding culture of the production organism at a given time. The non/low-producing isolate can typically be isolated following culture for >50-100 divisions (preferred), but a suitable isolate strain can also be genetically engineered by genetically inactivating one or several key heterologous production genes.

RNA-sequencing is carried out, preferably on at least three replicates, preferably using Illumina short-read sequencing according to standard methods known in the art.

Using standard bioinformatical analysis for differential gene expression e.g. incorporating the edgeR work-flow (DOI: 10.18129/B9.bioc.edgeR), suitable candidate burden-sensing promoters are identified from genes up-regulated by at least 5-10 times in the high-producing culture compared to the non/low-producing culture under the same typical production conditions. By identifying candidate burden-sensing promoters using this methodology, promoters activated preferentially by the burden of product formation (compared to merely the stress of production) can be found.

Next, in track 2, putative burden-sensing promoters (from track 1 or other lists) are confirmed using q-PCR on samples, similarly comparing one non/low-producer isolates to high-producer isolates during the same typical production conditions (in terms of e.g. temperature, stirring, product/by-product/substrate concentration/growth phase). Suitable candidate burden-sensing promoters are identified from genes up-regulated by at least 5-10 times in the high-producing culture compared to the non/low-producing culture under the same typical production conditions.

**Example 11: Burden addiction controlling multiple essential genes improves production of recombinant human growth hormone in *Escherichia coli***

The long-term production stability of the burden addicted *E. coli* strain s7.6#8 was improved by integration of one additional, different burden-sensing promoter controlling transcription of a second essential gene selected e.g. from the list and screened as suggested.

*11.1 Materials and methods*

s7.6#8 single colonies were transformed with a recombineering plasmid such as pKD46 using standard electroporation. Using recombineering, candidate burden-sensing promoters (Table 2) fused to variable RBSs (Table 3.1) were chromosomally integrated directly upstream of the essential gene *murI* or a similar essential gene, and selected for using spectinomycin resistance gene present in the integration constructs.

Following the described steps of recombineering and curing of the recombineering plasmid, five colonies were picked, corresponding to clones with the same promoter integration but having a random variant RBS sequence. Each clone was then transferred to a well of a 96-well plate containing 200 μl 2xYT supplemented with chloramphenicol for maintenance of the plasmid, pMevT, and cultured. 2 μl of each cultured clone was used to validate promotor integration as described in example 1.2.1; prior to freezing the 96-well plate.

*11.2 Results*

The different resulting clones were assessed for long-term production stability by serial passaging of 200x dilutions. Following long-term cultivation for approx. 75 cell generations, IgA-GFP production was quantified and shown to be improved in the new strains compared to the single burden addicted strains only featuring one burden-regulated essential gene.

**References**

Bonde, M. T., Pedersen, M., Klausen, M. S., Jensen, S. I., Wulff, T., Harrison, S., et al. (2016). Predictable tuning of protein expression in bacteria. Nat. Methods 13. doi:10.1038/nmeth.3727.

Giaever G et al., Nature. 2002 Jul 25;418(6896):387-91. DOI: 10.1038/nature00935 Jain, R., Kumar, P. & Varshney, U. A distinct role of formamidopyrimidine DNA glycosylase (MutM) in down-regulation of accumulation of G, C mutations and protection against oxidative stress in mycobacteria. DNA Repair (Amst). 6, 1774-1785 (2007). Guerout-Fleury AM, Frandson N, Stragier P. 1996. Plasmids for ectopic integration in Bacillus subtilis. Gene 180:57-61

Koskiniemi, S., Pränting, M., Gullberg, E., Näsvall, J., & Andersson, D. I. (2011). Activation of cryptic aminoglycoside resistance in Salmonella enterica. Molecular Microbiology, Vol. 80, pp. 1464-1478. https://doi.org/10.1111/j.1365-2958.2011.07657.x

Kimata, Y., Ishiwata-Kimata, Y., Yamada, S., and Kohno, K. (2006). Yeast unfolded protein response pathway regulates expression of genes for anti-oxidative stress and for cell surface proteins. Genes to Cells 11, 59-69. doi:10.1111/j.1365-2443.2005.00921.x.

Laferté, A., Favry, E., Sentenac, A., Riva, M., Carles, C., and Chédin, S. (2006). The transcriptional activity of RNA polymerase I is a key determinant for the level of all ribosome components. Genes Dev. 20, 2030-2040. doi:10.1101/gad.386106.

Maeda, M., Shimada, T., and Ishihama, A. (2015). Strength and Regulation of Seven rRNA Promoters in Escherichia

EP 4 137 571 B1

coli. PLoS One 10, 1-19.doi:10.1371/journal.pone.0144697.

Nonaka, G., Blankschien, M., Herman, C., Gross, C. a, and Rhodius, V. a (2006). Regulon and promoter analysis of the E. coli heat-shock factor, sigma32, reveals a multifaceted cellular response to heat stress. Genes Dev. 20, 1776-89. doi:10.1101/gad.1428206. Pitera, D. J., Paddon, C. J., Newman, J. D., and Keasling, J. D. (2007). Balancing a heterologous mevalonate pathway for improved isoprenoid production in Escherichia coli. Metab. Eng. 9, 193-207. doi:10.1016/j.ymben.2006.11.002.

Rugbjerg, P., Sarup-Lytzen, K., Nagy, M., and Sommer, M. O. A. (2018). Synthetic addiction extends the productive life time of engineered Escherichia coli populations. Proc. Natl. Acad. Sci. 115, 2347-2352. doi:10.1073/pnas.1718622115.

Wu, S. and Letchworth, G.J. (2004) High efficiency transformation by electroporation of Pichia pastoris pretreated with lithium acetate and dithiothreitol. Biotechniques 36, 152-154

Yoon, S. H., Han, M. J., Lee, S. Y., Jeong, K. J., and Yoo, J. S. (2003). Combined transcriptome and proteome analysis of Escherichia coli during high cell density culture. Biotechnol. Bioeng. 81, 753-767. doi:10.1002/bit.10626.

Uppal, S., Shetty, D. M., & Jawali, N. (2014). Cyclic AMP receptor protein regulates cspd, a bacterial toxin gene, in Escherichia coli. Journal of Bacteriology, 196(8), 1569-1577. https://doi.org/10.1128/JB.01476-13

Yamanaka, K., & Inouye, M. (1997). Growth-phase-dependent expression of cspD, encoding a member of the CspA family in Escherichia coli. Journal of Bacteriology, 179(16), 5126-5130. https://doi.org/10.1128/jb.179.16.5126-5130.1997

**Claims**

1. A microbial production cell genetically engineered to synthesize a product, said microbial cell further comprising:

    a. a first essential gene operably linked to a first burden-sensing promoter and
    b. a second essential gene operatively linked to a second burden-sensing promoter,
    wherein said first burden-sensing promoter is heterologous with respect to said first essential gene, and said second burden-sensing promoter is heterologous with respect to said second essential gene;
    wherein synthesis of the product confers a burden on said cell, and
    wherein expression of said first essential genes is up-regulated when said first burden-sensing promoter is induced by said burden relative to a basal level expression of said first essential gene when said first burden-sensing promoter is not induced, and expression of said second essential gene is up-regulated when said second burden-sensing promoter is induced by said burden relative to a basal level expression of said second essential gene when said second burden-sensing promoter is not induced.

2. The microbial production cell according to claim 1, wherein said burden conferred by synthesis of said product, when said first and second essential gene in the cell are operably linked to their native promoters, has a fitness cost measured as a percent reduction in the maximum exponential phase growth rate of the microbial production cell selected from among ≥ 5 %, ≥10%, ≥15%, ≥20%, ≥25%, ≥35%, and ≥ 45 % measured relative to a corresponding non-producing microbial cell.

3. The microbial production cell according to any one of claims 1 or 2, wherein the cell is:

    i. a bacterium belonging to a genus selected from among *Escherichia, Lactobacillus, Lactococcus, Corynebacterium, Bacillus, Acetobacter, Acinetobacter, Pseudomonas, Proprionibacterium, Bacteroides,* and *Bifidobacterium;* or
    ii. a yeast belonging to a genus selected from among *Saccharomyces, Kluyveromyces, Candida, Pichia, Komagataella, Cryptococcus, Debaromyces, Hansenula, Yarrowia, Zygosaccharomyces,* and *Schizosaccharomyces;* or
    iii. a filamentous fungus selected from among *Penicillium, Rhizopus, Fusarium, Fusidium, Gibberella, Mucor, Mortierella, Trichoderma Thermomyces, Streptomyces,* and *Aspergillus.*

4. The microbial production cell according to any one of claims 1-3, wherein the first and second essential genes are identical.

5. The microbial production cell according to any one of claims 1-4, wherein the first and second burden-sensing promoters are different.

6. The microbial production cell according to any one of claims 1-5, wherein the first and/or second essential gene is a non-conditional essential gene.

7. The microbial production cell according to any one of claims 1-6, wherein the first and/or second essential gene is operably linked to a synthetic RBS whose sequence is selected to modify the translational strength of the first and/or second essential gene independent of induction of said first and/or second burden-sensing promoter, respectively.

8. The microbial production cell according to any one of claims 1-7, wherein the first and second burden-sensing promoters are selected from among:

   i. a ribosomal RNA promoter,
   ii. a promoter upregulated by oxyR or a homolog thereof,
   iii. a promoter comprising a UPR element upregulated by HAC1 or a homolog thereof, and
   iv. a DNA damage-sensing promoter.

9. The microbial production cell according to any one of claims 1-8, wherein the cell is **characterized by** an increased product yield after at least 25, 30, 35, 40, 45, 50, 60, 70, 80, 90, or 100 generations of cell division from a single cell, as compared to a parent microbial production cell lacking said first and second essential genes operably linked to said first and second burden-sensing promoters, respectively.

10. The microbial production cell according to any one of claims 1-9, wherein the cell is **characterized by** an increased product yield of at least 10, 25, 50, or 80% following at least 50 generations of cell division from a single cell as compared to a parent microbial production cell lacking said first and second essential genes operably linked to said first and second burden-sensing promoters, respectively.

11. A method of product biosynthesis comprising the steps of:

   i. providing at least one microbial production cell according to any one of claims 1-10,
   ii. introducing the at least one cell into a cultivation medium comprising substrate for production of said product, and
   iii. recovering said product.

12. A method of product biosynthesis according to claim 11, wherein the product is selected from among: an organic acid, terpenoid, isoprenoid, polyketide, alcohol, sugar, vitamin, aldehyde, carboxylic acid, fatty acid, amino acid, peptide, enzyme (such as an amylase, lipase, protease, barnase, β-galactosidase, crystal protein, cutinase, PETase, laccase and carbohydrate active enzyme (such as a xylanase, lichenase, cellulase, lytic polysaccharide monooxoygenase, and pectase)), a therapeutic protein and a precursor thereof (such as human growth hormone, insulin, glucagon-like peptide-1, monoclonal- and polyclonal-antibody, single-fragment antibody and nanobody), a protein naturally found in eggs (such as ovalbumin), a milk protein (such as casein, lactadherin, lactoferrin), secreted immunoglobulin A and G, secretory components, and nanobodies.

13. Use of a first and a second essential gene operably linked to a first and a second burden-sensing promoter, respectively, to enhance product yield of a cultured population of microbial production cells arising from a single cell following at least 50 generations of cell division from said single cell as compared to a parent microbial production cell lacking said first and second essential genes operably linked to said first and second burden-sensing promoters, respectively;

   wherein said first burden-sensing promoter is heterologous with respect to said first essential gene, and said second burden-sensing promoter is heterologous with respect to said second essential gene;
   wherein synthesis of the product confers a burden on said cell; and
   wherein expression of said first essential gene is up-regulated when said first burden-sensing promoter is induced by said burden relative to a basal level expression of said first essential gene when said first burden-sensing promoter is not induced, and expression of said second essential gene is up-regulated when said second burden-sensing promoter is induced by said burden relative to a basal level expression of said second essential gene when said second burden-sensing promoter is not induced.

14. Use of said first and second essential gene operably linked to said first and second burden-sensing promoter, respectively, according to claim 13, wherein the product yield is enhanced by at least 10, 25, 50, or 80% following at least 50 generations of cell division from the single cell as compared to the parent microbial production cell lacking said first and second essential genes operably linked to said first and second burden-sensing promoters, respectively.

15. Use of a microbial production cell according to any one of claims 1-10 for producing a biosynthetic product, such as organic acids, terpenoids, isoprenoids, polyketides, alcohols, sugars, vitamins, aldehydes, carboxylic acids, fatty acids, amino acids, peptides, enzymes (such as amylases, lipases, proteases, barnases, β-galactosidases, crystal proteins, cutinases, PETases, laccases and carbohydrate active enzymes (such as xylanases, lichenases, cellulases, lytic polysaccharide monooxoygenases, and pectases)), therapeutic proteins and precursors thereof (such as human growth hormone, insulin, glucagon-like peptide-1, monoclonal and polyclonal antibodies, single-fragment antibodies), proteins naturally found in eggs (such as ovalbumin), milk proteins (such as caseins, lactadherins, lactoferrin), secreted immunoglobulin A and G, secretory components, and nanobodies.

## Patentansprüche

1. Mikrobielle Produktionszelle, welche gentechnisch verändert wurde, um ein Produkt zu synthetisieren, wobei die mikrobielle Zelle weiter umfasst:

   a) ein erstes essentielles Gen, welches mit einem ersten belastungsabhängigen Promotor operativ verknüpft ist, und

   b) ein zweites essentielles Gen, welches mit einem zweiten belastungsabhängigen Promotor operativ verknüpft ist,
   wobei der erste belastungsabhängige Promotor in Bezug auf das erste essentielle Gen heterolog ist und der zweite belastungsabhängige Promotor in Bezug auf das zweite essentielle Gen heterolog ist;
   wobei die Synthese des Produkts eine Belastung für die Zelle darstellt, und
   wobei Expression der ersten essentiellen Gene, wenn der erste belastungsabhängige Promotor durch die Belastung induziert wird, relativ zu einer Expression des ersten essentiellen Gens auf einem basalen Niveau hochreguliert wird, wenn der erste belastungsabhängige Promotor nicht induziert wird, und Expression des zweiten essentiellen Gens, wenn der zweite belastungsabhängige Promotor durch die Belastung induziert wird, relativ zu einer Expression des zweiten essentiellen Gens auf einem basalen Niveau hochreguliert wird, wenn der zweite belastungsabhängige Promotor nicht induziert wird.

2. Mikrobielle Produktionszelle nach Anspruch 1, wobei die Belastung durch die Synthese des Produkts, wenn das erste und zweite essentielle Gen in der Zelle mit ihren nativen Promotoren operativ verknüpft sind, Fitnesskosten aufweist, welche als prozentuale Verringerung der maximalen exponentiellen Wachstumsrate der mikrobiellen Produktionszelle gemessen werden, welche ausgewählt sind aus ≥5 %, ≥10 %, ≥15 %, ≥20 %, ≥25 %, ≥35 %, und ≥45 %, gemessen relativ zu einer entsprechenden nichtproduzierenden mikrobiellen Zelle.

3. Mikrobielle Produktionszelle nach einem der Ansprüche 1 oder 2, wobei die Zelle:

   i. ein Bakterium ist, welches zu einer Gattung gehört, welche ausgewählt ist aus *Escherichia, Lactobacillus, Lactococcus, Corynebakterium, Bazillus, Acetobacter, Acinetobacter, Pseudomonas, Proprionibacterium, Bacteroides* und *Bifidobakterium;* oder
   ii. eine Hefe ist, welche zu einer Gattung gehört, welche ausgewählt ist aus *Saccharomyces, Kluyveromyces, Candida, Pichia, Komagataella, Kryptokokken, Debaromyces, Hansenula, Yarrowia, Zygosaccharomyces* und *Schizosaccharomyces;* oder
   iii. ein Fadenpilz ist, welcher ausgewählt ist aus *Penicillium, Rhizopus, Fusarium, Fusidium, Gibberella, Mucor, Mortierella, Trichoderma Thermomyces, Streptomyceten* und *Aspergillus.*

4. Mikrobielle Produktionszelle nach einem der Ansprüche 1-3, wobei das erste und zweite essentielle Gen identisch sind.

5. Mikrobielle Produktionszelle nach einem der Ansprüche 1-4, wobei der erste und zweite belastungsabhängige Promotor unterschiedlich sind.

6. Mikrobielle Produktionszelle nach einem der Ansprüche 1-5, wobei das erste und/oder zweite essentielle Gen ein nicht bedingtes essentielles Gen ist.

7. Mikrobielle Produktionszelle nach einem der Ansprüche 1-6, wobei das erste und/oder zweite essentielle Gen mit einem synthetischen RBS operativ verknüpft ist, dessen Sequenz so ausgewählt ist, dass sie die Translationsstärke des ersten und/oder zweiten essentiellen Gens unabhängig von der Induktion des ersten und/oder zweiten belastungsabhängigen Promotors jeweils verändert.

8. Mikrobielle Produktionszelle nach einem der Ansprüche 1-7, wobei der erste und zweite belastungsabhängige Promotor ausgewählt sind aus:

    i. einem ribosomalen RNA-Promotor,
    ii. einem durch oxyR oder ein Homolog davon hochregulierten Promotor,
    iii. einem Promotor, welcher ein durch HAC1 oder ein Homolog davon hochreguliertes UPR-Element umfasst, und
    iv. einem DNA-schadenerkennenden Promotor.

9. Mikrobielle Produktionszelle nach einem der Ansprüche 1-8, wobei die Zelle durch eine erhöhte Produktausbeute nach zumindest 25, 30, 35, 40, 45, 50, 60, 70, 80, 90 oder 100 Generationen von Zellteilung aus einer einzelnen Zelle im Vergleich zu einer mikrobiellen Mutterproduktionszelle gekennzeichnet ist, welcher das erste und zweite essentielle Gen fehlen, welche jeweils mit den ersten und zweiten belastungsabhängige Promotoren operativ verknüpft sind.

10. Mikrobielle Produktionszelle nach einem der Ansprüche 1-9, wobei die Zelle durch eine erhöhte Produktausbeute von zumindest 10, 25, 50, oder 80 % nach zumindest 50 Generationen von Zellteilung aus einer einzelnen Zelle im Vergleich zu einer mikrobiellen Mutterproduktionszelle gekennzeichnet ist, welcher das erste und zweite essentielle Gen fehlen, welche jeweils mit den ersten und zweiten belastungsabhängige Promotoren operativ verknüpft sind.

11. Verfahren zur Produktbiosynthese, umfassend die folgenden Schritte von:

    i. Bereitstellen zumindest einer mikrobiellen Produktionszelle nach einem der Ansprüche 1-10,
    ii. Einbringen der zumindest einen Zelle in ein Kultivierungsmedium, welches ein Substrat zur Herstellung des Produkts umfasst, und
    iii. Wiederherstellen des Produkts.

12. Verfahren zur Produktbiosynthese nach Anspruch 11, wobei das Produkt ausgewählt ist aus: einer organischen Säure, einem Terpenoid, Isoprenoid, Polyketid, Alkohol, Zucker, Vitamin, Aldehyd, einer Carbonsäure, Fettsäure, Aminosäure, einem Peptid, Enzym (wie etwa einer Amylase, Lipase, Protease, Barnase, $\beta$-Galaktosidase, einem Kristallprotein, einer Kutinase, PETase, Laccase und einem kohlenhydrataktiven Enzym (wie etwa einer Xylanase, Lichenase, Zellulase, lytischen Polysaccharidmonooxoygenase und Pektase)), einem therapeutischen Protein und einem Vorläufer davon (wie etwa menschliches Wachstumshormon, Insulin, Glucagon-ähnliches Peptid-1, monoklonaler und polyklonaler Antikörper, Einzelfragment-Antikörper und Nanobody), einem natürlicherweise in Eiern vorkommenden Protein (wie etwa Ovalbumin), einem Milchprotein (wie etwa Casein, Lactadherin, Laktoferrin), sekretiertes Immunglobulin A und G, sekretorischen Komponenten und Nanobodys.

13. Verwendung eines ersten und eines zweiten essentiellen Gens, welche mit einem ersten bzw. einem zweiten belastungsabhängigen Promotor operativ verknüpft sind, um die Produktausbeute einer kultivierten Population mikrobieller Produktionszellen, welche aus einer einzelnen Zelle nach zumindest 50 Generationen der Zellteilung aus der einzelnen Zelle entstehen, im Vergleich zu einer mikrobiellen Mutterproduktionszelle zu erhöhen, welcher das erste und zweite essentielle Gen fehlen, welche jeweils mit den ersten und zweiten belastungsabhängige Promotoren operativ verknüpft sind;

    wobei der erste belastungsabhängige Promotor in Bezug auf das erste essentielle Gen heterolog ist und der zweite belastungsabhängige Promotor in Bezug auf das zweite essentielle Gen heterolog ist;
    wobei die Synthese des Produkts eine Belastung für die Zelle darstellt; und
    wobei Expression des ersten essentiellen Gens, wenn der erste belastungsabhängige Promotor durch die Belastung induziert wird, relativ zu einer Expression des ersten essentiellen Gens auf einem basalen Niveau hochreguliert wird, wenn der erste belastungsabhängige Promotor nicht induziert wird, und Expression des zweiten essentiellen Gens, wenn der zweite belastungsabhängige Promotor durch die Belastung induziert wird, relativ zu einer Expression des zweiten essentiellen Gens auf einem basalen Niveau hochreguliert wird, wenn der zweite belastungsabhängige Promotor nicht induziert wird.

**14.** Verwendung des ersten und zweiten essentiellen Gens, welche mit dem ersten bzw. zweiten belastungsabhängigen Promotor operativ verknüpft sind, nach Anspruch 13, wobei die Produktausbeute durch zumindest 10, 25, 50, oder 80 % nach zumindest 50 Generationen von Zellteilung aus der einzelnen Zelle im Vergleich zu der mikrobiellen Mutterproduktionszelle erhöht wird, welcher das erste und zweite essentielle Gen fehlen, welche jeweils mit den ersten und zweiten belastungsabhängige Promotoren operativ verknüpft sind.

**15.** Verwendung einer mikrobiellen Produktionszelle nach einem der Ansprüche 1-10 zur Herstellung eines biosynthetischen Produkts, wie etwa organische Säuren, Terpenoide, Isoprenoide, Polyketide, Alkohole, Zucker, Vitamine, Aldehyde, Carbonsäuren, Fettsäuren, Aminosäuren, Peptide, Enzyme (wie etwa Amylasen, Lipasen, Proteasen, Barnasen, β-Galaktosidasen, Kristallproteine, Kutinasen, PETasen, Laccasen und kohlenhydrataktive Enzyme (wie etwa Xylanasen, Lichenasen, Zellulasen, lytische Polysaccharidmonooxoygenasen und Pektasen)), therapeutische Proteine und Vorläufer davon (wie etwa menschliches Wachstumshormon, Insulin, Glucagon-ähnliches Peptid-1, monoklonale und polyklonale Antikörper, Einzelfragment-Antikörper), Proteine, welche natürlicherweise in Eiern vorkommen (wie etwa Ovalbumin), Milchproteine (wie etwa Caseine, Lactadherine, Laktoferrin), sekretiertes Immunglobulin A und G, Sekretkomponenten und Nanobodys.

## Revendications

**1.** Cellule de production microbienne génétiquement modifiée pour synthétiser un produit, ladite cellule microbienne comprenant en outre :

a) un premier gène essentiel lié fonctionnellement à un premier promoteur de détection de charge et
b) un second gène essentiel lié fonctionnellement à un second promoteur de détection de charge,
dans laquelle ledit premier promoteur de détection de charge est hétérologue par rapport audit premier gène essentiel, et ledit second promoteur de détection de charge est hétérologue par rapport audit second gène essentiel ;
dans laquelle la synthèse du produit confère une charge à ladite cellule, et
dans laquelle l'expression dudit premier gène essentiel est régulée à la hausse lorsque ledit premier promoteur de détection de charge est induit par ladite charge par rapport à une expression de niveau basal dudit premier gène essentiel lorsque ledit premier promoteur de détection de charge n'est pas induit, et l'expression dudit second gène essentiel est régulée à la hausse lorsque ledit second promoteur de détection de charge est induit par ladite charge par rapport à une expression de niveau basal dudit second gène essentiel lorsque ledit second promoteur de détection de charge n'est pas induit.

**2.** Cellule de production microbienne selon la revendication 1, dans laquelle ladite charge conférée par la synthèse dudit produit, lorsque lesdits premier et second gènes essentiels dans la cellule sont liés fonctionnellement à leurs promoteurs natifs, présente un coût d'adaptation mesuré en tant que pourcentage de réduction du taux de croissance de phase exponentielle maximal de la cellule de production microbienne sélectionné parmi ≥ 5 %, ≥ 10 %, ≥ 15 %, ≥ 20 %, ≥ 25 %, ≥ 35 %, et ≥ 45 % mesuré par rapport à une cellule microbienne non productrice correspondante.

**3.** Cellule de production microbienne selon l'une quelconque des revendications 1 ou 2, dans laquelle la cellule est :

i. une bactérie appartenant à un genre choisi parmi *Escherichia, Lactobacillus, Lactococcus, Corynebacterium, Bacillus, Acetobacter, Acinetobacter, Pseudomonas, Proprionibacterium, Bacteroides* et *Bifidobacterium* ; ou
ii. une levure appartenant à un genre choisi parmi *Saccharomyces, Kluyveromyces, Candida, Pichia, Komagataella, Cryptococcus, Debaromyces, Hansenula, Yarrowia, Zygosaccharomyces* et *Schizosaccharomyces* ; ou
iii. un champignon filamenteux choisi parmi *Penicillium, Rhizopus, Fusarium, Fusidium, Gibberella, Mucor, Mortierella, Trichoderma, Thermomyces, Streptomyces* et *Aspergillus.*

**4.** Cellule de production microbienne selon l'une quelconque des revendications 1-3, dans laquelle les premier et second gènes essentiels sont identiques.

**5.** Cellule de production microbienne selon l'une quelconque des revendications 1-4, dans laquelle les premier et second promoteurs de détection de charge sont différents.

**6.** Cellule de production microbienne selon l'une quelconque des revendications 1-5, dans laquelle le premier et/ou second gène essentiel est un gène essentiel non conditionnel.

7. Cellule de production microbienne selon l'une quelconque des revendications 1-6, dans laquelle le premier et/ou second gène essentiel est lié fonctionnellement à un RBS synthétique dont la séquence est sélectionnée pour modifier la force de traduction du premier et/ou second gène essentiel indépendant de l'induction dudit premier et/ou second promoteur de détection de charge, respectivement.

8. Cellule de production microbienne selon l'une quelconque des revendications 1-7, dans laquelle les premier et second promoteurs de détection de charge sont choisis parmi :

   i. un promoteur d'ARN ribosomique,
   ii. un promoteur régulé à la hausse par oxyR ou un homologue de celui-ci,
   iii. un promoteur comprenant un élément UPR régulé à la hausse par HAC1 ou un homologue de celui-ci, et
   iv. un promoteur de détection des dommages à l'ADN.

9. Cellule de production microbienne selon l'une quelconque des revendications 1-8, dans laquelle la cellule est **caractérisée par** un rendement de produit accru après au moins 25, 30, 35, 40, 45, 50, 60, 70, 80, 90 ou 100 générations de division cellulaire à partir d'une seule cellule, par rapport à une cellule de production microbienne parente dépourvue desdits premier et second gènes essentiels liés fonctionnellement auxdits premier et second promoteurs de détection de charge, respectivement.

10. Cellule de production microbienne selon l'une quelconque des revendications 1-9, dans laquelle la cellule est **caractérisée par** un rendement de produit accru d'au moins 10, 25, 50 ou 80 % après au moins 50 générations de division cellulaire à partir d'une seule cellule, par rapport à une cellule de production microbienne parente dépourvue desdits premier et second gènes essentiels liés fonctionnellement auxdits premier et second promoteurs de détection de charge, respectivement.

11. Procédé de biosynthèse de produit comprenant les étapes de :

    i. fourniture d'au moins une cellule de production microbienne selon l'une quelconque des revendications 1-10,
    ii. introduction de la au moins une cellule dans un milieu de culture comprenant un substrat pour la production dudit produit, et
    iii. récupération dudit produit.

12. Procédé de biosynthèse de produit selon la revendication 11, dans lequel le produit est choisi parmi : un acide organique, un terpénoïde, un isoprénoïde, un polycétide, un alcool, un sucre, une vitamine, un aldéhyde, un acide carboxylique, un acide gras, un acide aminé, un peptide, une enzyme (telle qu'une amylase, une lipase, une protéase, une barnase, une β-galactosidase, une protéine cristalline, une cutinase, une PETase, une laccase et une enzyme active sur les glucides (telle qu'une xylanase, une lichénase, une cellulase, une monooxygénase polysaccharidique lytique et une pectase)), une protéine thérapeutique et un précurseur de celle-ci (tel que l'hormone de croissance humaine, l'insuline, le peptide-1 de type glucagon, un anticorps monoclonal et polyclonal, un anticorps à fragment unique et un nanocorps), une protéine naturellement présente dans les oeufs (telle que l'ovalbumine), une protéine du lait (telle que la caséine, la lactadhérine, la lactoferrine), des immunoglobulines A et G sécrétées, des composants sécrétoires, et des nanocorps.

13. Utilisation d'un premier et d'un second gène essentiel liés fonctionnellement à un premier et à un second promoteur de détection de charge, respectivement, pour améliorer le rendement de produit d'une population cultivée de cellules de production microbienne issues d'une cellule unique après au moins 50 générations de division cellulaire à partir de ladite cellule unique par rapport à une cellule de production microbienne parente dépourvue desdits premier et second gènes essentiels liés fonctionnellement auxdits premier et second promoteurs de détection de charge, respectivement ;

    dans laquelle ledit premier promoteur de détection de charge est hétérologue par rapport audit premier gène essentiel, et ledit second promoteur de détection de charge est hétérologue par rapport audit second gène essentiel ;
    dans laquelle la synthèse du produit confère une charge à ladite cellule ; et
    dans laquelle l'expression dudit premier gène essentiel est régulée à la hausse lorsque ledit premier promoteur de détection de charge est induit par ladite charge par rapport à une expression de niveau basal dudit premier gène essentiel lorsque ledit premier promoteur de détection de charge n'est pas induit, et l'expression dudit second gène essentiel est régulée à la hausse lorsque ledit second promoteur de détection de charge est induit par ladite charge par rapport à une expression de niveau basal dudit second gène essentiel lorsque ledit second promoteur de détection de charge n'est pas induit.

14. Utilisation desdits premier et second gènes essentiels liés fonctionnellement auxdits premier et second promoteurs de détection de charge, respectivement, selon la revendication 13, dans laquelle le rendement de produit est amélioré d'au moins 10, 25, 50 ou 80 % après au moins 50 générations de division cellulaire à partir de la cellule unique par rapport à la cellule de production microbienne parente dépourvue desdits premier et second gènes essentiels liés fonctionnellement auxdits premier et second promoteurs de détection de charge, respectivement.

15. Utilisation d'une cellule de production microbienne selon l'une quelconque des revendications 1-10 pour produire un produit biosynthétique, tel que des acides organiques, des terpénoïdes, des isoprénoïdes, des polycétides, des alcools, des sucres, des vitamines, des aldéhydes, des acides carboxyliques, des acides gras, des acides aminés, des peptides, des enzymes (telles que des amylases, des lipases, des protéases, des barnases, des $\beta$-galactosidases, des protéines cristallines, des cutinases, des PETases, des laccases et des enzymes actives sur les glucides (telles que des xylanases, des lichénases, des cellulases, des monooxygénases polysaccharidiques lytiques et des pectases)), des protéines thérapeutiques et des précurseurs de celles-ci (tels que l'hormone de croissance humaine, l'insuline, le peptide-1 de type glucagon, des anticorps monoclonaux et polyclonaux, des anticorps à fragment unique), des protéines naturellement présentes dans les oeufs (telles que l'ovalbumine), des protéines du lait (telles que les caséines, les lactadhérines, la lactoferrine), des immunoglobulines A et G sécrétées, des composants sécrétoires et des nanocorps.

**Figure 1**

**Figure 2**

**Figure 3**

**Figure 4**

**Figure 5**

**Figure 6**

**Figure 7**

**Figure 8**

**Figure 9**

**Figure 10**

**Figure 11**

**Figure 12**

**Figure 13**

**Figure 14 A**

**Figure 14 B**

**Figure 15**

IgA-GFP production (500x dilution seeds)

**Figure 16**

IgA-GFP production (500x dilution seeds)

**Figure 17**

Production in double burden-sensing strains
following five seeds of serial passage

**Figure 18**

IgA-GFP production (after five 500x
dilution seeds measured by ELISA)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **RUGBJERG et al.** *PNAS,* 2018
- **BONDE, M. T. ; PEDERSEN, M. ; KLAUSEN, M. S ; JENSEN, S. I. ; WULFF, T. ; HARRISON, S. et al.** Predictable tuning of protein expression in bacteria. *Nat. Methods,* 2016, vol. 13
- **GIAEVER G et al.** *Nature,* 25 July 2002, vol. 418 (6896), 387-91
- **JAIN, R. ; KUMAR, P. ; VARSHNEY, U.** A distinct role of formamidopyrimidine DNA glycosylase (MutM) in down-regulation of accumulation of G, C mutations and protection against oxidative stress in mycobacteria. *DNA Repair (Amst).,* 2007, vol. 6, 1774-1785
- **GUEROUT-FLEURY AM ; FRANDSON N ; STRAGIER P.** Plasmids for ectopic integration in Bacillus subtilis. *Gene,* 1996, vol. 180, 57-61
- **KOSKINIEMI, S. ; PRÄNTING, M ; GULLBERG, E. ; NÄSVALL, J. ; ANDERSSON, D. I.** Activation of cryptic aminoglycoside resistance in Salmonella enterica. *Molecular Microbiology,* 2011, vol. 80, 1464-1478, https://doi.org/10.1111/j.1365-2958.2011.07657.x
- **KIMATA, Y. ; ISHIWATA-KIMATA, Y. ; YAMADA, S. ; KOHNO, K.** Yeast unfolded protein response pathway regulates expression of genes for anti-oxidative stress and for cell surface proteins. *Genes to Cells,* 2006, vol. 11, 59-69
- **LAFERTÉ, A. ; FAVRY, E. ; SENTENAC, A. ; RIVA, M. ; CARLES, C. ; CHÉDIN, S.** The transcriptional activity of RNA polymerase I is a key determinant for the level of all ribosome components. *Genes Dev.,* 2006, vol. 20, 2030-2040
- **MAEDA, M ; SHIMADA, T ; ISHIHAMA, A.** Strength and Regulation of Seven rRNA Promoters in Escherichia coli. *PLoS One,* 2015, vol. 10, 1-19

- **NONAKA, G. ; BLANKSCHIEN, M ; HERMAN, C. ; GROSS, C. A ; RHODIUS, V. A.** Regulon and promoter analysis of the E. coli heat-shock factor, sigma32, reveals a multifaceted cellular response to heat stress. *Genes Dev.,* 2006, vol. 20, 1776-89
- **PITERA, D. J. ; PADDON, C. J. ; NEWMAN, J. D. ; KEASLING, J. D.** Balancing a heterologous mevalonate pathway for improved isoprenoid production in Escherichia coli. *. Metab. Eng.,* 2007, vol. 9, 193-207
- **RUGBJERG, P ; SARUP-LYTZEN, K. ; NAGY, M. ; SOMMER, M. O. A.** Synthetic addiction extends the productive life time of engineered Escherichia coli populations. *Proc. Natl. Acad. Sci.,* 2018, vol. 115, 2347-2352
- **WU, S. ; LETCHWORTH, G.J.** High efficiency transformation by electroporation of Pichia pastoris pretreated with lithium acetate and dithiothreitol. *Biotechniques,* 2004, vol. 36, 152-154
- **YOON, S. H ; HAN, M. J. ; LEE, S. Y. ; JEONG, K. J. ; YOO, J. S.** Combined transcriptome and proteome analysis of Escherichia coli during high cell density culture. *Biotechnol. Bioeng.,* 2003, vol. 81, 753-767
- **UPPAL, S ; SHETTY, D. M. ; JAWALI, N.** Cyclic AMP receptor protein regulates cspd, a bacterial toxin gene, in Escherichia coli. *Journal of Bacteriology,* 2014, vol. 196 (8), 1569-1577, https://doi.org/10.1128/JB.01476-13
- **YAMANAKA, K ; INOUYE, M.** Growth-phase-dependent expression of cspD, encoding a member of the CspA family in Escherichia coli. *Journal of Bacteriology,* 1997, vol. 179 (16), 5126-5130, https://doi.org/10.1128/jb.179.16.5126-5130.1997